# EUROPEAN PATENT SPECIFICATION

(11) **EP 1 753 476 B1**
(45) Date of publication and mention of the grant of the patent: **29.07.2015**
(21) Application number: 05747612.9
(22) Date of filing: 10.03.2005
(51) Int. Cl.: A61L 31/10, A61L 31/14, A61L 31/16, A61F 2/852, A61F 2/86

(54) **PROGENITOR ENDOTHELIAL CELL CAPTURING WITH A DRUG ELUTING IMPLANTABLE MEDICAL DEVICE**
VORLÄUFER-ENDOTHELZELL-ERFASSUNG MIT EINEM ARZNEIMITTEL ABGEBENDEN IMPLANTIERBAREN MEDIZINPRODUKT
CAPTURE DE CELLULES ENDOTHELIALES PROGENITRICES AU MOYEN D'UN DISPOSITIF MEDICAL IMPLANTABLE A ELUTION MEDICAMENTEUSE

(30) Priority: 10.03.2004 US 551978 P
(43) Date of publication of application: 21.02.2007
(60) Divisional application: 13180123.5
(73) Proprietor: OrbusNeich Medical, Inc., Ft Lauderdale, FL 33309 (US)
(72) Inventor: COTTONE, Robert, John, Jr., Davie, FL 33330 (US); ROWLAND, Stephen, M., Miami, FL 33157 (US); DAVIS, Horace, R., Coral Springs, FL 33071 (US); KUTRYK, Michael, J., B., York, Ontario M5P 3J7 (CA)
(74) Representative: Jilderda, Anne Ayolt
(86) International application number: PCT/US2005/007654
(87) International publication number: WO 2005/086831

(56) References cited:
- WO-A1-03/099169
- WO-A2-03/065881
- CA-A1- 2 260 532
- US-A- 4 553 974
- US-A- 5 464 450
- US-B1- 6 171 609
- US-B2- 6 541 116
- US-B2- 6 613 083

## Description

This application claims benefit of U.S. Provisional Application Serial No. 60/551,978, filed on March 10, 2004.

### TECHNICAL FIELD OF INVENTION

The invention relates to a medical device for implantation into vessels or luminal structures within the body. More particularly, the present invention relates to stents and synthetic grafts which are coated with a controlled-release matrix comprising a medicinal substance for direct delivery to the surrounding tissues, and a ligand attached thereto for capturing progenitor endothelial cells in the blood contacting surface of the device to form mature endothelium at site of injury. In particular, the polymer matrix/drug/ligand-coated stents are for use, for example, in therapy of diseases such as restenosis, artherosclerosis, and endoluminal reconstructive therapies.

### BACKGROUND OF INVENTION

Atherosclerosis is one of the leading causes of death and disability in the world. Atherosclerosis involves the deposition of fatty plaques on the luminal surface of arteries. The deposition of fatty plaques on the luminal surface of the artery causes narrowing of the cross-sectional area of the artery. Ultimately, this deposition blocks blood flow distal to the lesion causing ischemic damage to the tissues supplied by the artery.

Coronary arteries supply the heart with blood. Coronary artery atherosclerosis disease (CAD) is the most common, serious, chronic, life-threatening illness in the United States, affecting more than 11 million persons. The social and economic costs of coronary atherosclerosis vastly exceed that of most other diseases. Narrowing of the coronary artery lumen causes destruction of heart muscle resulting first in angina, followed by myocardial infarction and finally death. There are over 1.5 million myocardial infarctions in the United States each year. Six hundred thousand (or 40%) of those patients suffer an acute myocardial infarction and more than three hundred thousand of those patients die before reaching the hospital. (Harrison's Principles of Internal Medicine,14th Edition, 1998).

CAD can be treated using percutaneous transluminal coronary balloon angioplasty (PTCA). More than 400,000 PTCA procedures are performed each year in the United States. In PTCA, a balloon catheter is inserted into a peripheral artery and threaded through the arterial system into the blocked coronary artery. The balloon is then inflated, the artery stretched, and the obstructing fatty plaque flattened, thereby increasing the cross-sectional flow of blood through the affected artery. The therapy, however, does not usually result in a permanent opening of the affected coronary artery. As many as 50% of the patients who are treated by PTCA require a repeat procedure within six months to correct a re-narrowing of the coronary artery. Medically, this re-narrowing of the artery after treatment by PTCA is called restenosis.

Acutely, restenosis involves recoil and shrinkage of the vessel. Subsequently, recoil and shrinkage of the vessel are followed by proliferation of medial smooth muscle cells in response to injury of the artery from PTCA. In response to blood vessel injury, smooth muscle cells in the tunica media and fibroblasts of the adventitial layer undergo phenotypic change which results in the secretion of metalloproteases into the surrounding matrix, luminal migration, proliferation and protein secretion. Various other inflammatory factors are also released into the injured area including thromboxane A₂, platelet derived growth factor (PDGF) and fibroblast growth factor (FGF). A number of different techniques have been used to overcome the problem of restenosis, including treatment of patients with various pharmacological agents or mechanically holding the artery open with a stent. (Harrison's Principles of Internal Medicine, 14th Edition, 1998). Initial attempts at preventative therapy, that targeted smooth muscle cell proliferation, proved ineffective. It has become apparent that to be effective earlier events in the restenotic process must be targeted, and subsequent approaches focused on the inhibition of cell regulatory pathways using genetic therapies. Unfortunately, none of these therapies have shown promise for the prevention of restenosis. This lack of success of molecular techniques has led to a revival in the interest of conventional pharmacotherapeutic approaches.

Of the various procedures used to overcome restenosis, stents have proven to be the most effective. Stents are metal scaffolds that are positioned in the diseased vessel segment to create a normal vessel lumen. Placement of the stent in the affected arterial segment prevents recoil and subsequent closing of the artery. Stents can also prevent local dissection of the artery along the medial layer of the artery. By maintaining a larger lumen than that created using PTCA alone, stents reduce restenosis by as much as 30%. Despite their success, stents have not eliminated restenosis entirely. (Suryapranata et al. 1998. Randomized comparison of coronary stenting with balloon angioplasty in selected patients with acute myocardial infarction. Circulation 97:2502-2502).

Narrowing of the arteries can occur in vessels other than the coronary arteries, including the aortoiliac, infrainguinal, distal profunda femoris, distal popliteal, tibial, subclavian and mesenteric arteries. The prevalence of peripheral artery atherosclerosis disease (PAD) depends on the particular anatomic site affected as well as the criteria used for diagnosis of the occlusion. Traditionally, physicians have used the test of intermittent claudication to determine whether PAD is present. However, this measure may vastly underestimate the actual incidence of the disease in the population. Rates of PAD appear to vary with age, with an increasing incidence of PAD in older individuals. Data from the National Hospital Discharge Survey estimate that every year, 55,000 men and 44,000 women had a first-listed diagnosis of chronic PAD and 60,000 men and 50,000 women had a first-listed diagnosis of acute PAD. Ninety-one percent of the acute PAD cases involved the lower extremity. The prevalence of comorbid CAD in patients with PAD can exceed 50%. In addition, there is an increased prevalence of cerebrovascular disease among patients with PAD.

PAD can be treated using percutaneous translumenal balloon angioplasty (PTA). The use of stents in conjunction with PTA decreases the incidence of restenosis. However, the post-operative results obtained with medical devices such as stents do not match the results obtained using standard operative revascularization procedures, i.e., those using a venous or prosthetic bypass material. (Principles of Surgery, Schwartz et al. eds., Chapter 20, Arterial Disease, 7th Edition, McGraw-Hill Health Professions Division, New York 1999).

Preferably, PAD is treated using bypass procedures where the blocked section of the artery is bypassed using a graft. (Principles of Surgery, Schwartz et al. eds., Chapter 20, Arterial Disease, 7th Edition, McGraw-Hill Health Professions Division, New York 1999). The graft can consist of an autologous venous segment such as the saphenous vein or a synthetic graft such as one made of polyester, polytetrafluoroethylene (PTFE), or expanded polytetrafluoroethylene (ePTFE). The post-operative patency rates depend on a number of different factors, including the luminal dimensions of the bypass graft, the type of synthetic material used for the graft and the site of outflow. Restenosis and thrombosis, however, remain significant problems even with the use of bypass grafts. For example, the patency of infrainguinal bypass procedures at 3 years using an ePTFE bypass graft is 54% for a femoral-popliteal bypass and only 12% for a femoral-tibial bypass.

Consequently, there is a significant need to improve the performance of both stents and synthetic bypass grafts in order to further reduce the morbidity and mortality of CAD and PAD.

With stents, the approach has been to coat the stents with various antithrombotic or anti-restenotic agents in order to reduce thrombosis and restenosis. For example, impregnating stents with radioactive material appears to inhibit restenosis by inhibiting migration and proliferation of myofibroblasts. (U.S. Patent Nos. 5,059,166, 5,199,939 and 5,302,168). Irradiation of the treated vessel can pose safety problems for the physician and the patient. In addition, irradiation does not permit uniform treatment of the affected vessel.

Alternatively, stents have also been coated with chemical agents such as heparin or phosphorylcholine, both of which appear to decrease thrombosis and restenosis. Although heparin and phosphorylcholine appear to markedly reduce restenosis in animal models in the short term, treatment with these agents appears to have no long-term effect on preventing restenosis. Additionally, heparin can induce thrombocytopenia, leading to severe thromboembolic complications such as stroke. Therefore, it is not feasible to load stents with sufficient therapeutically effective quantities of either heparin or phosphorylcholine to make treatment of restenosis in this manner practical.

Synthetic grafts have been treated in a variety of ways to reduce postoperative restenosis and thrombosis. (Bos et al. 1998. Small-Diameter Vascular Graft Prostheses:Current Status Archives Physio. Biochem. 106:100-115). For example, composites of polyurethane such as meshed polycarbonate urethane have been reported to reduce restenosis as compared with ePTFE grafts. The surface of the graft has also been modified using radiofrequency glow discharge to add polyterephalate to the ePTFE graft. Synthetic grafts have also been impregnated with biomolecules such as collagen.

The endothelial cell (EC) layer is a crucial component of the normal vascular wall, providing an interface between the bloodstream and the surrounding tissue of the blood vessel wall. Endothelial cells are also involved in physiological events including angiogenesis, inflammation and the prevention of thrombosis (Rodgers GM. FASEB J 1988;2:116-123.). In addition to the endothelial cells that compose the vasculature, recent studies have revealed that ECs and progenitor endothelial cells (PECs) circulate postnatally in the peripheral blood (Asahara T, et al. Science 1997;275:964-7; Yin AH, et al. Blood 1997;90:5002-5012; Shi Q, et al. Blood 1998;92:362-367; Gehling UM, et al. Blood 2000;95:3106-3112; Lin Y, et al. J Clin Invest 2000;105:71-77). PECs are believed to migrate to regions of the circulatory system with an injured endothelial lining, including sites of traumatic and ischemic injury (Takahashi T, et al. Nat Med 1999;5:434-438). In normal adults, the concentration of EPCs in peripheral blood is 3-10 cells/mm³ (Takahashi T, et al. Nat Med 1999;5:434-438; Kalka C, et al. Ann Thorac Surg. 2000;70:829-834). It is now evident that each phase of the vascular response to injury is influenced (if not controlled) by the endothelium. It is believed that the rapid reestablishment of a functional endothelial layer on damaged stented vascular segments may help to prevent these potentially serious complications by providing a barrier to circulating cytokines, peventing adverse effects of a thrombus, and by the ability of endothelial cells to produce substances that passivate the underlying smooth muscle cell layer. (Van Belle et al. 1997. Stent Endothelialization. Circulation 95:438-448; Bos et al. 1998. Small-Diameter Vascular Graft Prostheses:Current Status Archives Physio. Biochem. 106:100-115).

Endothelial cells have been encouraged to grow on the surface of stents by local delivery of vascular endothelial growth factor (VEGF), an endothelial cell mitogen, after implantation of the stent (Van Belle et al. 1997. Stent Endothelialization. Circulation 95:438-448.). While the application of a recombinant protein growth factor, VEGF in saline solution at the site of injury induces desirable effects, the VEGF is delivered to the site of injury after stent implantation using a channel balloon catheter. This technique is not desirable since it has demonstrated that the efficiency of a single dose delivery is low and produces inconsistent results. Therefore, this procedure cannot be reproduced accurately every time.

Synthetic grafts have also been seeded with endothelial cells, but the clinical results with endothelial seeding have been generally poor, i.e., low post-operative patency rates (Lio et al. 1998. New concepts and Materials in Microvascular Grafting: Prosthetic Graft Endothelial Cell Seeding and Gene Therapy. Microsurgery 18:263-256) due most likely to the fact the cells did not adhere properly to the graft and/or lost their EC function due to *ex-vivo* manipulation.

Endothelial cell growth factors and environmental conditions *in situ* are therefore essential in modulating endothelial cell adherence, growth and differentiation at the site of blood vessel injury. Accordingly, with respect to restenosis and other blood vessel diseases, there is a need for the development of new methods and compositions for coating medical devices, including stents and synthetic grafts, which would promote and accelerate the formation of a functional endothelium on the surface of implanted devices so that a confluent EC monolayer is formed on the target blood vessel segment or grafted lumen and inhibiting neo-intimal hyperplasia.

U.S. Patents Nos. 5,288,711; 5,563,146; 5,516,781, and 5,646,160 disclose a method of treating hyperproliferative vascular disease with rapamycin alone or in combination with mycophenolic acid. The rapamycin is given to the patient by various methods including, orally, parenterally, intravascular, intranasally, intrabronchially, transdermally, rectally, etc. The patents further disclose that the rapamycin can be provided to the patient via a vascular stent, which is impregnated with the rapamycin alone or in combination with heparin or mycophenolic acid. One of the problems encountered with the impregnated stent of the patents is that the drug is released immediately upon contact with the tissue and does not last for the amount of time required to prevent restenosis.

The patent WO 03/099169 discloses a stent comprising a coating for controlled release of one or more pharmaceutical substances to adjacent tissue for inhibiting restenosis, wherein the coating comprises a bio-absorbable matrix and one or more pharmaceutical substances. The bioabsorbable matrix comprises one or more polymers or oligomers selected from the group consisting of poly (lactide- co-glycolide), polylactic acid, polyglycolic acid, a polyanhydride, polycaprolactone, polyhydroxybutyrate valerate.

European Patent Application No. EP 0 950 386 discloses a stent with local rapamycin delivery, in which the rapamycin is deliver to the tissues directly from micropores in the stent body, or the rapamycin is mixed or bound to a polymer coating applied on the stent. EP 0 950 386 further discloses that the polymer coating consists of purely nonabsorbable polymers such as polydimethylsiloxane, poly(ethylene-vingylacetate), acrylate based polymers or copolymers, etc. Since the polymers are purely nonabsorbable, after the drug is delivered to the tissues, the polymers remain at the site of implantation.

Nonabsorbable polymers remaining in large amounts adjacent to the tissues, however, have been known to induce inflammatory reactions on their own with restenosis recurring at the implantation site thereafter.

Additionally, U.S. Patent No. 5,997,517 discloses a medical device coated with a thick coherent bond coat of acrylics, epoxies, acetals, ethylene copolymers, vinyl polymers and polymers containing reactive groups. The polymers disclosed in the patent are also nonabsorbable and can cause side effects when used in implantable medical devices similarly as discussed above with respect to EP 0 950 386.

None of the aforementioned approaches has significantly reduced the incidence of thrombosis or restenosis over an extended period of time. Additionally, the coating of prior art medical devices have been shown to crack upon implantation of the devices. Therefore, new devices and methods of treatment are needed to treat vascular disease.

### SUMMARY OF INVENTION

The present invention provides a medical device for implanting into the lumen of a blood vessel or a organ with a luman according to claim 1.

The pharmaceutical substance or composition comprise one or more drugs or substances which inhibit smooth muscle cell migration and proliferation at the site of implantation. Additionally, the capturing of the progenitor endothelial cells on the luminal surface of the medical device accelerates the formation of a functional endothelium at the site of injury.

In one embodiment, the implantable medical device comprises a stent. The stent can be selected from uncoated stents available in the art. In accordance with one embodiment, the stent is an expandable intraluminal endoprosthesis comprising a tubular member as described in U.S. Patent Application US2004039441.

In another embodiment, the stent is made of a biodegradable material.

The controlled-release matrix comprises one or more polymers and/or oligomers from various types and sources, including, natural or synthetic polymers, which are biocompatible, biodegradable, bioabsorbable and useful for controlled-released of the medicament. For example, the synthetic material can include polyesters such as polylactic acid, polyglycolic acid or copolymers and or combinations thereof, a polyanhydride , polycaprolactone, polyhydroxybutyrate valerate, and other biodegradable polymer, or mixtures or copolymers thereof. In another embodiment, the naturally occurring polymeric materials can include proteins such as collagen, fibrin, elastin, and extracellular matrix component, or other biologic agents or mixtures thereof. The polymer material or mixture thereof can be applied together as a composition with the pharmaceutical substance on the surface of the medical device and can comprise a single layer. Multiple layers of composition can be applied to form the coating. In another embodiment, multiple layers of polymer material or mixtures thereof can be applied between layers of the pharmaceutical substance. For example, the layers may be applied sequentially, with the first layer directly in contact with the uncoated surface of the device and a second layer comprising the pharmaceutical substance and having one surface in contact with the first layer and the opposite surface in contact with a third layer of polymer which is in contact with the surrounding tissue. Additional layers of the polymer material and drug composition can be added as required, alternating each component or mixtures of components thereof.

Alternatively, the pharmaceutical substance can be applied as multiple layers of a composition and each layer can comprise one or more drugs surrounded by polymer material. In this embodiment, the multiple layers of pharmaceutical substance can comprise a pharmaceutical composition comprising multiple layers of a single drug; one or more drugs in each layer, and/or differing drug compositions in alternating layers applied. In one embodiment, the layers comprising pharmaceutical substance can be separated from one another by a layer of polymer material. In another embodiment, a layer of pharmaceutical composition may be provided to the device for immediate release of the pharmaceutical substance after implantation.

In another embodiment, the matrix comprises poly(lactide-coglycolide) as the matrix polymer for coating the medical device. In this embodiment of the invention, the poly(lactide-co-glycolide) composition comprises at least one polymer of poly-DL-co-glycolide or copolymer or mixtures thereof, and it is mixed together with the pharmaceutical substances to be delivered to the tissues. The coating composition is then applied to the surface of the device using standard techniques such as spraying, dipping, and/or chemical vaporization. Alternatively, the poly(lactide-co-glycolide)(PGLA) solution can be applied as a single layer separating a layer or layers of the pharmaceutical substance(s).

In another embodiment, the coating composition further comprises pharmaceutically acceptable polymers and/or pharmaceutically acceptable carriers, for example, nonabsorbable polymers, such as ethylene vinyl acetate (EVAC) and methylmethacrylate (MMA). The nonabsorbable polymer, for example, can aid in further controlling release of the substance by increasing the molecular weight of the composition thereby delaying or slowing the rate of release of the pharmaceutical substance.

Compounds or pharmaceutical compositions which can be incorporated in the matrix, include, but are not limited to immunosuppressant drugs such as rapamycin, drugs which inhibit smooth muscle cell migration and/or proliferation, antithrombotic drugs such as thrombin inhibitors, immunomodulators such as platelet factor 4 and CXC-chemokine; inhibitors of the CX3CR1 receptor family; antiinflammatory drugs, steroids such as dihydroepiandrosterone (DHEA), testosterone, estrogens such as 17β-estradiol; statins such as simvastatin and fluvastatin; PPAR-alpha and PPAR-gamma agonists such as rosglitazone; nuclear factors such as NF-κβ, collagen synthesis inhibitors, vasodialators, growth factors which induce endothelial cell growth and differentiation such as basic fibroblast growth factor (bFGF), platelet-derived growth factor, endothelial cell growth factor (EGF), vascular endothelial cell growth factor (VEGF); protein tyrosine kinase inhibitors such as Midostaurin and imatinib or any anti-angionesis inhibitor compound; peptides or antibodies which inhibit mature leukocyte adhesion, antibiotics/antimicrobials, and other substances such as butyric acid and butyric acid derivatives puerarin, fibronectin, and the like.

The coating on the device further comprises a ligand such as an antibody. The ligand can comprise a molecule which binds to a structure on the surface of cells such as progenitor endothelial cells, for example, at least one type of antibody, fragment of an antibody or combinations of antibody and fragments. In this aspect of the invention, the antibody can be a monoclonal antibody, a polyclonal antibody, a chimeric antibody, or a humanized antibody. The antibody or antibody fragment recognizes and binds a progenitor endothelial (endothelial cells, progenitor or stem cells with the capacity to become mature, functional endothelial cells) cell surface antigen and modulates the adherence of the cells onto the surface of the medical device. The antibody or antibody fragment of the invention can be covalently or noncovalently attached to the surface of the matrix, or tethered covalently by a linker molecule to the outermost layer of the matrix coating the medical device. In this aspect of the invention, for example, the monoclonal antibodies can further comprises Fab or F(ab')₂ fragments. The antibody fragment of the invention comprises any fragment size, such as large and small molecules which retain the characteristic to recognize and bind the target antigen as the antibody.

The antibodies and/or antibody fragmens recognize and bind with high affinity and specificity to antigens or molecules on the cell membrane surface of the circulating cells of the mammal being treated, and their specificity is not dependent on cell lineage. In one embodiment, for example, the antibody and/or fragment is specific for a human progenitor endothelial cell surface antigen such as CD133, CD34, CDw90, CD117, HLA-DR, VEGFR-1, VEGFR-2, Muc-18 (CD146), CD130, stem cell antigen (Sca-1), stem cell factor 1 (SCF/c-Kit ligand), Tie-2 and HAD-DR.

In another embodiment, the coating of the medical device comprises at least one layer of a biocompatible matrix as described above, the matrix comprising an outer surface for attaching a therapeutically effective amount of at least one type of small molecule of natural or synthetic origin. The small molecule recognizes and interacts with an antigen on cell such as a progenitor endothelial cell surface to immobilize the progenitor endothelial cell on the surface of the device to form endothelium. The small molecules can be derived from a variety of sources such as cellular components such as fatty acids, peptides, proteins, nucleic acids, saccharides and the like and can interact, for example, with a structure such as an antigen on the surface of a progenitor endothelial cell with the same results or effects as an antibody.

In another embodiment, there is provided a method for treating vascular disease such as restenosis and artherosclerosis, comprising administering a pharmaceutical substance locally to a patient in need of such substance. The method comprises implanting into a vessel or hollowed organ of a patient a medical device with a coating, which coating comprises a pharmaceutical composition comprising a drug or substance for inhibiting smooth muscle cell migration and thereby restenosis, and a biocompatible, biodegradable, bioerodible, nontoxic polymer matrix, such as poly(lactide-co-glycolide) copolymer, or mixtures thereof, wherein the pharmaceutical composition comprises a slow or controlled-release formulation for the delayed release of the drug. The coating on the medical device can also comprise a ligand such as an antibody for capturing cells such as endothelial cells and or progenitor cells on the luminal surface of the device so that a functional endothelium is formed.

In another embodiment, there is provided a method of making a coated medical device or a medical device with a coating, which comprises applying to the medical device a pharmaceutical composition comprising a biocompatible, biodegradable, nontoxic polymer matrix such as poly(lactide-co-glycolide) copolymer, and one or more pharmaceutical substances, wherein the matrix and the substance(s) are mixed prior to applying to the medical device and thereafter, applying a solution comprising at least one type of ligand to the surface of the device on top or on the outer surface of the device with the drug/matrix composition. The method may alternatively comprise the step of applying at least one layer of a pharmaceutical composition comprising one or more drugs and pharmaceutically acceptable carriers, and applying at least one layer of a polymer matrix to the medical device. In the method, the polymer matrix with or without the pharmaceutical substance, and the ligand can be applied independently to the medical device by several methods using standard techniques, such as dipping, spraying or vapor deposition. In an alternate embodiment, the polymer matrix can be applied to the device with or without the pharmaceutical substance. In this aspect of the invention wherein the polymer matrix is applied without the drug, the drug is applied as a layer between layers of matrices.

In one embodiment, the method comprises applying the pharmaceutical composition as multiple layers with the ligand applied on the outermost surface of the medical device so that, for example, the ligand such as antibodies can be attached in the luminal surface of the device.

In another embodiment, the coating is comprised of a multiple component pharmaceutical matrix such as a fast release pharmaceutical agent to retard early neointimal hyperplasia/smooth muscle cell migration and a secondary biostable matrix that releases a long term agent for maintaining vessel patency such as endothelial nitric oxide synthase (eNOS), tissue plasminogen activator, statins, steroids, and/or antibiotics.

### BRIEF DESCRIPTION OF DRAWINGS

FIG. 1 is a schematic representation of an embodiment in which a stent strut comprises a coating surrounding the entire device and consisting of a ligand (outer) layer, a drug/polymer matrix (inner) layer surrounding the entire circumference of the strut.
FIG. 2 is a schematic representation of an embodiment in which a stent strut comprises a ligand (outer) layer and a drug/polymer layer surrounding about three quarters of the circumference of the strut.
FIG. 3 is a schematic representation of an embodiment in which a stent strut comprises a ligand (outer) layer and a drug/polymer layer surrounds three quarters of the circumference of the strut and drug/polymer concentration is greater in the middle section of the layer surrounding the strut.
FIG. 4 is a schematic representation of an embodiment in which a stent strut comprises a ligand (outer) layer and a drug/polymer layer is applied in a section of the circumference of the strut and which appears as half circles in cross-section.
FIG. 5 is a schematic representation of an embodiment in which a stent strut comprises a ligand (outer) layer and a drug/polymer layer applied to a section of the circumference of the strut.
FIG. 6 is a schematic representation of an embodiment in which a stent strut comprises a ligand layer which is applied on the entire circumference of the strut and a drug/polymer layer is applied in dot matrix like pattern to a portion of the strut.
FIG. 7 is a schematic representation of an embodiment in which a stent strut comprises a drug/polymer layer surrounding the circumference of the strut and a ligand layer is applied on top of the drug/polymer layer, and an additional drug/polymer composition is applied on a portion of strut's surface in a dot matrix like pattern.
FIG. 8A and 8B are schematic representations of alternate embodiments in which a stent strut comprises a ligand layer is applied to the entire circumference of the strut and a drug/polymer layer composition is applied on a portion of strut's surface in a dot matrix like pattern on top of the ligand layer (8A), and a drug/polymer matrix in a dot matrix like pattern is applied on the surface of the device and a ligand layer surrounding the entire circumference of the strut and covering the drug/polymer composition (8B).
FIG. 9 is a schematic representation of an embodiment in which a stent strut is shown in cross-section showing multiple layers of the coating including ligand (antibody) and drug/polymer components.
FIG. 10A is a schematic representation of an embodiment in which a stent strut is shown in cross-section showing multiple layers of the coating including intermediate and basement membrane layers on the surface of the strut. FIG. 10B is a schematic representation of an embodiment in which a stent's component parts, i.e., helices, rings and ends are coated with different coating components.
FIG. 11 is a schematic representation of a stent partially coated to show the drug eluting composition and the ligand layer.
FIG. 12 is a schematic representation of a cross-section of a stent showing the layers of the coating.
FIG. 13 is a graph showing the elution profile of a drug-coated stent, incubated for 21 days in bovine serum albumin, wherein the coating comprised 500 µg of 4% Paclitaxel and 96% polymer. The polymer used in the coating was 50:50 Poly(DL Lactide-co-Glycolide).
FIG. 14 is a graph showing the elution profile of a drug-coated stent, incubated for 10 days in bovine serum albumin, wherein the coating comprised 500 µg of 8% Paclitaxel and 92% polymer. The polymer used in the coating was 50:50 Poly(DL Lactide-co-Glycolide)/EVAC 25.
FIG. 15 is a graph showing the drug elution profile of a drug-coated stent incubated for 10 days in bovine serum, wherein the coating comprised 500 µg of 8% Paclitaxel and 92% polymer. The polymer used in the coating was 80:20 Poly-DL Lactide/EVAC 25.
FIG. 16 is a graph showing the drug elution profile of a drug-coated stent, incubated for 21 days in bovine serum albumin, wherein the coating comprised 500 µg of 8% Paclitaxel and 92% poly(DL-Lactide) polymer.
FIG. 17 is a graph showing the elution profile of drug-coated stent incubated for 1, 14, and 28 days in serum albumin, wherein the coating comprised Paclitaxel and PGLA.
FIG. 18 is a graph showing drug elution test results of a stent coated with 4% Paclitaxel in 96% PGLA polymer matrix and in 100% PGLA incubated in serum albumin for up to 70 days.
FIGs. 19A-19D are photographs of drug-coated stents after 90 days (FIGs. 19A and 19B) and 84 days (FIGs. 19C and 19D) after incubation on serum albumin.
FIGs. 20A-21E are photomicrographs of HUVECs attached to carboxymethyldextran (CMDx) and anti-CD34 antibody (20A); gelatin and anti-CD34 antibody (20B); bare stainless steel disc (20C); CMDx coated and gelatin coated stainless steel disc which were incubated with HUVEC cell and stained with propidium iodide.
FIGs. 21A-21C are photomicrographs of a control stainless steel discs, coated with CMDx without antibody. FIGs. 21D-21F are photomicrographs of control stainless steel discs coated with gelatin without antibody bound to its surface.
FIGs. 22A-22C are photomicrographs of stainless steel discs coated with CMDx matrix with anti-CD34 antibody bound to its surface. FIGs. 22D-22F are photomicrographs of stainless steel discs coated with gelatin matrix with antibody bound to its surface.

### DETAILED DESCRIPTION

In embodiments illustrated herein, there is provided a medical device in the form of an implantable structure, which is coated with a homogenous matrix comprising a pharmaceutical substance distributed in a biodegradable, biocompatible, non-toxic, bioerodible, bioabsorbable polymer matrix, as described in U.S. Application Serial No. 10/442,669, and a ligand such as an antibody or any other suitable molecule attached to the matrix for capturing and immobilizing circulating cells such as endothelial and progenitor endothelial cells on the luminal surface of the device. The medical device provides a mechanism for rapidly forming a functional endothelium at the site of implantation of the device, as described in pending U.S. Applications US20040551978 and US2003229393.

The structure of the medical device has at least one surface and comprises at least one or more base materials and it is for implanting into the lumen of an organ or a blood vessel. The based materials can be of various types, for example, stainless steel, Nitinol, MP35N, gold, tantalum, platinum or platinum iridium, or other biocompatible metals and/or alloys such as carbon or carbon fiber, cellulose acetate, cellulose nitrate, silicone, cross-linked polyvinyl acetate (PVA) hydrogel, cross-linked PVA hydrogel foam, polyurethane, polyamide, styrene isobutylene-styrene block copolymer (Kraton), polyethylene teraphthalate, polyurethane, polyamide, polyester, polyorthoester, polyanhidride, polyether sulfone, polycarbonate, polypropylene, high molecular weight polyethylene, polytetrafluoroethylene, or other biocompatible polymeric material, or mixture of copolymers thereof; polyesters such as, polylactic acid, polyglycolic acid or copolymers thereof, a polyanhydride, polycaprolactone, polyhydroxybutyrate valerate or other biodegradable polymer, or mixtures or copolymers, extracellular matrix components, proteins, collagen, fibrin or other bioactive agent, or mixtures thereof.

The medical device can be any device that is introduced temporarily or permanently into a mammal for the prophylaxis or therapy of a medical condition. These devices include any that are introduced subcutaneously, percutaneously or surgically to rest within an organ, tissue or lumen of an organ, such as arteries, veins, ventricles and/or atrium of the heart. Medical devices may include stents, stent grafts; covered stents such as those covered with polytetrafluoroethylene (PTFE), expanded polytetrafluoroethylene (ePTFE), or synthetic vascular grafts, artificial heart valves, artificial hearts and fixtures to connect the prosthetic organ to the vascular circulation, venous valves, abdominal aortic aneurysm (AAA) grafts, inferior venal caval filters, permanent drug infusion catheters, embolic coils, embolic materials used in vascular embolization (e.g., cross-linked PVA hydrogel), vascular sutures, vascular anastomosis fixtures, transmyocardial revascularization stents and/or other conduits.

The coating composition on the medical device comprises one or more pharmaceutical substances incorporated into a polymer matrix so that the pharmaceutical substance(s) is released locally into the adjacent or surrounding tissue in a slow or controlled-release manner and one or more ligands attached to the blood contacting surface of the medical device. The release of the pharmaceutical substance in a controlled manner allows for smaller amounts of drug or active agent to be released for a long period of time in a zero order elution profile manner. The release kinetics of a drug further depends on the hydrophobicity of the drug, i.e., the more hydrophobic the drug is, the slower the rate of release of the drug from the matrix. Alternative, hydrophilic drugs are released from the matrix at a faster rate. Therefore, the matrix composition can be altered according to the drug to be delivered in order to maintain the concentration of drug required at the implantation site for a longer period of time. There is, therefore, provided a long term effect of the drugs at the required site which may be more efficient in preventing restenosis and which may minimize the side effects of the released pharmaceutical substances used.

The matrix can comprise a variety of polymer matrices. However, the matrix should be biocompatible, biodegradable, bioerodible, non-toxic, bioabsorbable, and with a slow rate of degradation. Biocompatible matrices that can be used in the invention include, but are not limited to, poly(lactide-co-glycolide), polyesters such as polylactic acid, polyglycolic acid or copolymers thereof, polyanhydride, polycaprolactone, polyhydroxybutyrate valerate, and other biodegradable polymer, or mixtures or copolymers, and the like. In another embodiment, the naturally occurring polymeric materials can be selected from proteins such as collagen, fibrin, elastin, and extracellular matrix components, or other biologic agents or mixtures thereof.

Polymer matrices which can be used in the coating can include polymers such as poly(lactide-co-glycolide); poly-DL-lactide, poly-L-lactide, and/or mixtures thereof and can be of various inherent viscosities and molecular weights. For example, in one embodiment, poly(DL lactide-co-glycolide) (DLPLG, Birmingham Polymers Inc.) can be used. Poly(DL-lactide-co-glycolide) is a bioabsorbable, biocompatible, biodegradable, non-toxic, bioerodible material, which is a vinylic monomer and can serve as a polymeric colloidal drug carrier. The poly-DL-lactide material can be in the form of homogeneous composition and when solubilized and dried, it can form a lattice of channels in which pharmaceutical substances can be trapped for delivery to the tissues.

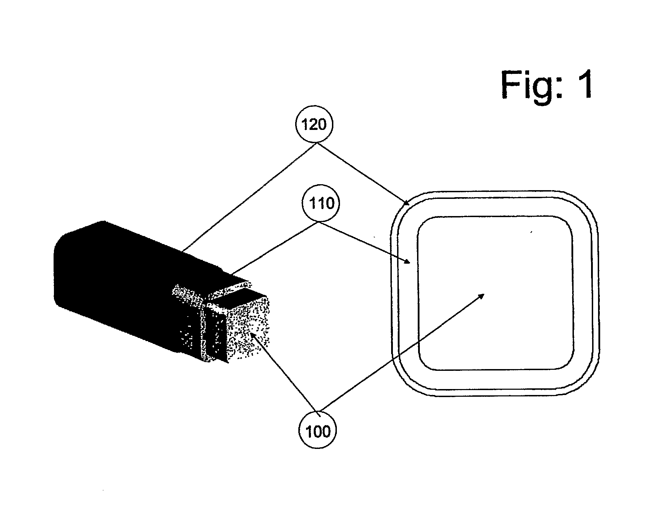

The drug release kinetics of the coating on the device can also be controlled depending on the inherent viscosity of the polymer or copolymer used as the matrix, and the amount of drug in the composition. The polymer or copolymer characteristics can vary depending on the inherent viscosity of the polymer or copolymer. For example, in one embodiment wherein poly(DL-lactide-co-glycolide)is used, the inherent viscosity can range from about 0.55 to about 0.75 (dL/g). Poly(DL-Lactide-co-Glycolide) can be added to the coating composition from about 50 to about 99% (w/w) of the polymeric composition. FIG. 1 is illustrative of a stent partially coated with the coating comprising poly(DL-lactide-co-glycolide) polymer matrix. The poly(DL-lactide-co-glycolide) polymer coating deforms without cracking, for example, when the coated medical device is subjected to stretch and/or elongation and undergoes plastic and/or elastic deformation. Therefore, polymers which can withstand plastic and elastic deformation such as poly(DL-lactide-co-glycolide) acid-based coats have advantageous characteristics over prior art polymers. Furthermore, the rate of dissolution of the matrix can also be controlled by using polymers of various molecular weight. For example, for slower rate of release of the pharmaceutical substances, the polymer should be of higher molecular weight. By varying the molecular weight of the polymer or combinations thereof, a preferred rate of dissolution can be achieved for a specific drug. Alternatively, the rate of release of pharmaceutical substances can be controlled by applying a polymer layer to the medical device, followed by one or more layers of drug(s), followed by one or more layers of the polymer. Additionally, polymer layers can be applied between drug layers to decrease the rate of release of the pharmaceutical substance from the coating.

The malleability of the coating composition can be further modified by varying the ratio of lactide to glycolide in the copolymer. For example, the ratio of components of the polymer can be adjusted to make the coating more malleable and to enhance the mechanical adherence of the coating to the surface of the medical device and aid in the release kinetics of the coating composition. In this embodiment, the polymer can vary in molecular weight depending on the rate of drug release desired. The ratio of lactide to glycolide can range, respectively, from about 50-85% to about 50-15% in the composition. By adjusting the amount of, for example, lactide in the polymer, the rate of release of the drugs from the coating can also be controlled.

The characteristic biodegradation of the polymer, therefore, can determine the rate of drug release from the coating. Information on the biodegradation of polymers can be obtained from the manufacturer information, for example, for lactides from Birmingham Polymers.

The principle mode of degradation, for example, for lactide and glycolide polymers and copolymers is hydrolysis. Degradation proceeds first by diffusion of water into the material followed by random hydrolysis, fragmentation of the material and finally a more extensive hydrolysis accompanied by phagocytosis, diffusion and metabolism. The hydrolysis of the material is affected by the size and hydrophillicity of the particular polymer, the crystallinity of the polymer and the pH and temperature of the environment.

In one embodiment, the degradation time may be shorter, for example, for low molecular weight polymers, more hydrophillic polymers, more amorphous polymers and copolymers higher in glycolide. Therefore at identical conditions, low molecular weight copolymers of DL-Lactide and Glycolide, such as 50/50 DL-PLG can degrade relatively rapidly whereas the higher molecular weight homopolymers such as L-PLA may degrade much more slowly.

Once the polymer is hydrolyzed, the products of hydrolysis are either metabolized or secreted. Lactic acid generated by the hydrolytic degradation of, for example, PLA can become incorporated into the tricarboxylic acid cycle and can be secreted as carbon dioxide and water. PGA can also be broken down by random hydrolysis accompanied by non-specific enzymatic hydrolysis to glycolic acid which can be either secreted or enzymatically converted to other metabolized species.

In another embodiment, the coating composition comprises a nonabsorbable polymer, such as ethylene vinyl acetate (EVAC), polybutyl-methacrylate (PBMA) and methylmethacrylate (MMA) in amounts from about 0.5 to about 99% of the final composition. The addition of EVAC, PBMA or methylmethacrylate can further increase malleability of the matrix so that the device can be more plastically deformable. The addition of methylmethacrylate to the coating can delay the degradation of the coat and therefore, can also improve the controlled release of the coat so that the pharmaceutical substance is released at even slower rates.

The coating of the medical device can be applied to the medical device using standard techniques to cover the entire surface of the device, or partially, as a single layer of a homogeneous mixture of drugs and matrix, or in a composition in a dot matrix pattern. In embodiments wherein the matrix and/or matrix/drug composition is applied as a single or multiple layers, the matrix or composition is applied in a thickness of from about 0.1 µm to about 150 µm; or from about 1 µm to about 100 µm. Alternative, multiple layers of the matrix/drug composition can be applied on the surface of the device in this thickness range. For example, multiple layers of various pharmaceutical substances can be deposited onto the surface of the medical device so that a particular drug can be released at one time, one drug in each layer, which can be separated by polymer matrix. The active ingredient or pharmaceutical substance component of the composition can range from about 1% to about 60% (w/w) or the composition. Upon contact of the coating composition with adjacent tissue where implanted, the coating can begin to degrade in a controlled manner. As the coating degrades, the drug is slowly released into adjacent tissue and the drug is eluted from the device so that the drug can have its effect locally. Additionally, since the polymers used with the device can form a lattice of channels, the drugs can be released slowly from the channels upon implantation of the device. The coated medical device provides an improved and local mechanism for delivering a drug to surrounding tissue without affecting the patient systemically. The drug elution via channels in the coating matrix and degradation of the matrix can be accomplished so that drug(s) can elute from the surface of the medical device once implanted for about a period from about one week to about one year. The drug may elute by erosion as well as diffusion when drug concentrations are low. With high concentrations of drug, the drug may elute via channels in the coating matrix.

The pharmaceutical substance of the invention includes drugs which are used in the treatment of vascular disease, such as artherosclerosis and restenosis. For example, the pharmaceutical substances include, but are not limited to antibiotics/antimicrobials, antiproliferatives, antineoplastics, antioxidants, endothelial cell growth factors, thrombin inhibitors, immunosuppressants, anti-platelet aggregation agents, collagen synthesis inhibitors, therapeutic antibodies, nitric oxide donors, antisense oligonucleotides, wound healing agents, therapeutic gene transfer constructs, peptides, proteins, extracellular matrix components, vasodialators, thrombolytics, anti-metabolites, growth factor agonists, antimitotics, statins, steroids, steroidal and nonsterodial antiinflammatory agents, angiotensin converting enzyme (ACE) inhibitors, free radical scavengers, PPAR-gamma agonists, anti-cancer chemotherapeutic agents. For example, some of the aforementioned pharmaceutical substances include, cyclosporins A (CSA), rapamycin, rapamycin derivatives, mycophenolic acid (MPA), retinoic acid, n-butyric acid, butyric acid derivatives, vitamin E, probucol, L-arginine-L-glutamate, everolimus, sirolimus, biolimus, biolimus A-9, paclitaxel, puerarin, platelet factor 4, basic fibroblast growth factor (bFGF), fibronectin, simvastatin, fluvastatin, dihydroepiandrosterone (DHEA), and 17ß-estradiol.

FIGs. 1-10 show schematic representation of various embodiments of the coating of the present medical device. The coating on the medical device comprising a biocompatible matrix for promoting the formation of a confluent layer of functional endothelial cells on the luminal surface of the device and pharmaceutical substances which inhibit excessive intimal smooth muscle cell hyperplasia, and thereby preventing restenosis and thrombosis. In one embodiment, the matrix comprises a synthetic or naturally-occurring material in which a therapeutically effective amount of at least one type of molecule such as an antibody that promotes adherence of endothelial, progenitor or stem cells to the medical device, and at least one compound such as a rapamycin, rapamycin derivatives, and/or estradiol for delivering to adjacent tissues. Upon implantation of the device, the cells that adhere to the surface of the device transform into a mature, confluent, functional layer of endothelium on the luminal surface of the medical device. The presence of a confluent layer of endothelial cells on the medical device can reduce the occurrence of restenosis and thrombosis at the site of implantation.

As used herein, "medical device" refers to a device that is introduced temporarily or permanently into a mammal for the prophylaxis or therapy of a medical condition. These devices include any that are introduced subcutaneously, percutaneously or surgically to rest within an organ, tissue or lumen of an organ, such as arteries, veins, ventricles or atrium of the heart. Medical devices may include stents, stent grafts, covered stents such as those covered with polytetrafluoroethylene (PTFE), expanded polytetrafluoroethylene (ePTFE), or synthetic vascular grafts, artificial heart valves, artificial hearts and fixtures to connect the prosthetic organ to the vascular circulation, venous valves, abdominal aortic aneurysm (AAA) grafts, inferior venal caval filters, permanent drug infusion catheters, embolic coils, embolic materials used in vascular embolization (e.g., cross-linked PVA hydrogel), vascular sutures, vascular anastomosis fixtures, transmyocardial revascularization stents and/or other conduits. In one embodiment, the stent can be made from a biodegradable material.

Coating of the medical device with the compositions and methods can stimulate the development of a confluent endothelial cell monolayer on the surface of the medical device as well as can modulate local chronic inflammatory response and other thromboembolic complications that result from blood vessel injury during implantation of the medical device.

As used herein, the term "antibody" refers to one type of antibody such as monoclonal, polyclonal, humanized, or chimeric antibody or a combination thereof, and wherein the monoclonal, polyclonal, humanized or chimeric antibody has high affinity and specificity for binding to one antigen or a functional equivalent of that antigen or other structure on the surface of the target cell. The term antibody fragment encompasses any fragment of an antibody such as Fab, F(ab')₂, and can be of any size, i.e., large or small molecules, which have the same results or effects as the antibody. (An antibody encompasses a plurality of individual antibody molecules equal to 6.022 x 10²³ molecules per mole of antibody).

In an aspect of the invention, a stent or synthetic graft of the invention is coated with a biocompatible, controlled-release matrix comprising antibodies that modulate adherence of circulating progenitor endothelial cells to the medical device. The antibodies of the invention recognize and bind with high affinity and specificity to progenitor endothelial cells surface antigens in the circulating blood so that the cells are immobilized on the surface of the device. In one embodiment, the antibodies comprise monoclonal antibodies reactive (recognize and bind) with progenitor endothelial cell surface antigens, or a progenitor or stem cell surface antigen, such as vascular endothelial growth factor receptor-1, -2 and -3 (VEGFR-1, VEGFR-2 and VEGFR-3 and VEGFR receptor family isoforms), Tie-1, Tie2, CD34, Thy-1, Thy-2, Muc-18 (CD146), CD30, stem cell antigen-1 (Sca-1), stem cell factor (SCF or c-Kit ligand), CD133 antigen, VE-cadherin, P1H12, TEK, CD31, Ang-1, Ang-2, or an antigen expressed on the surface of progenitor endothelial cells. In one embodiment, a single type of antibody that reacts with one antigen can be used. Alternatively, a plurality of different types of antibodies directed against different progenitor endothelial cell surface antigens can be mixed together and added to the matrix. In another embodiment, a cocktail of monoclonal antibodies is used to increase the rate of epithelium formation by targeting specific cell surface antigens. In this aspect of the invention, for example, anti-CD34 and anti-CD133 are used in combination and attached to the surface of the matrix on a stent or graft.

As used herein, a "therapeutically effective amount of the antibody" means the amount of an antibody that promotes adherence of endothelial, progenitor or stem cells to the medical device. The amount of an antibody needed to practice the invention varies with the nature of the antibody used. For example, the amount of an antibody used depends on the binding constant between the antibody and the antigen against which it reacts. It is well known to those of ordinary skill in the art how to determine therapeutically effective amounts of an antibody to use with a particular antigen.

As used herein, "intimal hyperplasia" is the undesirable increased in smooth muscle cell proliferation and matrix deposition in the vessel wall. As used herein "restenosis" refers to the reoccurrent narrowing of the blood vessel lumen. Vessels may become obstructed because of restenosis. After PTCA or PTA, smooth muscle cells from the media and adventitia, which are not normally present in the intima, proliferate and migrate to the intima and secrete proteins, forming an accumulation of smooth muscle cells and matrix protein within the intima. This accumulation causes a narrowing of the lumen of the artery, reducing blood flow distal to the narrowing. As used herein, "inhibition of restenosis" refers to the inhibition of migration and proliferation of smooth muscle cells accompanied by prevention of protein secretion so as to prevent restenosis and the complications arising therefrom.

The subjects that can be treated using the medical device, methods and compositions of this invention are mammals, and include a human, horse, dog, cat, pig, rodent, monkey and the like.

The term "progenitor endothelial cell" refers to endothelial cells at any developmental stage, from progenitor or stem cells to mature, functional epithelial cells from bone marrow, blood or local tissue origin and which are non-malignant.

For *in vitro* studies or use of the coated medical device, fully differentiated endothelial cells may be isolated from an artery or vein such as a human umbilical vein, while progenitor endothelial cells are isolated from peripheral blood or bone marrow. The endothelial cells are bound to the medical devices by incubation of the endothelial cells with a medical device coated with the matrix that incorporates an antibody, a growth factor, or other agent that adheres to endothelial cells. In another embodiment, the endothelial cells can be transformed endothelial cells. The transfected endothelial cells contain vectors which express growth factors or proteins which inhibit thrombogenesis, smooth muscle cell migration, restenosis, or any other therapeutic end.

The methods of treatment of vascular disease illustrated herein can be practiced on any artery or vein. Included within the scope of this invention is atherosclerosis of any artery including coronary, infrainguinal, aortoiliac, subclavian, mesenteric and renal arteries. Other types of vessel obstructions, such as those resulting from a dissecting aneurysm are also encompassed by the invention.

The method of treating a mammal with vascular disease comprises implanting a coated medical device into the patient's organ or vessel, for example, in the case of a coated stent during angioplastic surgery. Once *in situ,* progenitor endothelial cells are captured on the surface of the coated stent by the recognition and binding of antigens on the progenitor cell surface by the antibody present on the coating. Once the progenitor cell is adhered to the matrix, the growth factor on the coating promotes the newly-bound progenitor endothelial cells to grow and differentiate and form a confluent, mature and functional endothelium on the luminal surface of the stent. Alternatively, the medical device is coated with the endothelial cells *in vitro* before implantation of the medical device using progenitor, stem cells, or mature endothelial cells isolated from the patient's blood, bone marrow, or blood vessel. In either case, the presence of endothelial cells on the luminal surface of the medical device inhibits or prevents excessive intimal hyperplasia and thrombosis.

Human umbilical vein endothelial cells (HUVEC) are obtained from umbilical cords according to the methods of Jaffe, et al., J. Clin. Invest., 52:2745-2757, 1973. Briefly, cells are stripped from the blood vessel walls by treatment with collagenase and cultured in gelatin-coated tissue culture flasks in M199 medium containing 10% low endotoxin fetal calf serum, 90 ug/ml preservative-free porcine heparin, 20 ug/ml endothelial cell growth supplement (ECGS) and glutamine.

Progenitor endothelial cells (EPC) are isolated from human peripheral blood according to the methods of Asahara et al. (Isolation of putative progenitor endothelial cells for angiogenesis. Science 275:964-967, 1997. Magnetic beads coated with antibody to CD34 are incubated with fractionated human peripheral blood. After incubation, bound cells are eluted and can be cultured in EBM-2 culture medium. (Clonetics, San Diego, CA). Alternatively enriched medium isolation can be used to isolate these cells. Briefly, peripheral venous blood is taken from healthy male volunteers and the mononuclear cell fraction is isolated by density gradient centrifugation, and the cells are plated on fibronectin coated culture slides (Becton Dickinson) in EC basal medium-2 (EBM-2) (Clonetics) supplemented with 5% fetal bovine serum, human VEGF-A, human fibroblast growth factor-2, human epidermal growth factor, insulin-like growth factor-1, and ascorbic acid. EPCs are grown for 7-days, with culture media changes every 48 hours. Cells are characterized by fluorescent antibodies to CD133, CD45, CD34, CD31, VEGFR-2, Tie-2, and E-selectin.

As used herein "ligand" refers to a molecule that binds a cell membrane structure such as a receptor molecule on the circulating endothelial and/or progenitor cell. For example, the ligand can be an antibody, antibody fragment, small molecules such as peptides, cell adhesion molecule, basement membrane component, such as basement membrane proteins, for example, elastin, fibrin, cell adhesion molecules, and fibronectin. In an embodiment using antibodies, the antibodies recognize and bind a specific epitope or structure, such as cell surface receptor on the cell membrane of the cell.

In one embodiment, the antibodies are monoclonal antibodies and may be produced according to the standard techniques of Kohler and Milstein (Continuous cultures of fused cells secreting antibody of predefined specificity. Nature 265:495-497, 1975), or can be obtained from commercial sources. Endothelial cells can be used as the immunogen to produce monoclonal antibodies directed against endothelial cell surface antigens.

In this aspect of the invention, the monoclonal antibodies directed against endothelial cells may be prepared by injecting HUVEC or purified progenitor endothelial cells into a mouse or rat. After a sufficient time, the mouse is sacrificed and spleen cells are obtained. The spleen cells are immortalized by fusing them with myeloma cells or with lymphoma cells, generally in the presence of a non-ionic detergent, for example, polyethylene glycol. The resulting cells, which include the fused hybridomas, are allowed to grow in a selective medium, such as HAT-medium, and the surviving cells are grown in such medium using limiting dilution conditions. The cells are grown in a suitable container, e.g., microtiter wells, and the supernatant is screened for monoclonal antibodies having the desired specificity, i.e., reactivity with endothelial cell antigens.

Various techniques exist for enhancing yields of monoclonal antibodies such as injection of the hybridoma cells into the peritoneal cavity of a mammalian host which accepts the cells and then harvesting the ascites fluid. Where an insufficient amount of monoclonal antibody collects in the ascites fluid, the antibody is harvested from the blood of the host. Various conventional ways exist for isolation and purification of monoclonal antibodies so as to free the monoclonal antibodies from other proteins and other contaminants.

Also included within the scope of the invention are useful binding fragments of anti-endothelial cell monoclonal antibodies such as the Fab, F(ab')₂ of these monoclonal antibodies. The antibody fragments are obtained by conventional techniques. For example, useful binding fragments may be prepared by peptidase digestion of the antibody using papain or pepsin.

Antibodies of the invention are directed to an antibody of the IgG class from a murine source; however, this is not meant to be a limitation. The above antibody and those antibodies having functional equivalency with the above antibody, whether from a murine source, mammalian source including human, or other sources, or combinations thereof are included within the scope of this invention, as well as other classes such as IgM, IgA, IgE, and the like, including isotypes within such classes. In the case of antibodies, the term "functional equivalency" means that two different antibodies each bind to the same antigenic site on an antigen, in other words, the antibodies compete for binding to the same antigen. The antigen may be on the same or different molecule.

In one embodiment, monoclonal antibodies reacting with the endothelial cell surface antigen CD34, and/or CD133 are used. Anti-CD34 monoclonal antibodies attached to a solid support have been shown to capture progenitor endothelial cells from human peripheral blood. After capture, these progenitor cells are capable of differentiating into endothelial cells. (Asahara et al. 1997. Isolation of putative progenitor endothelial cells for angiogenesis. Science 275:964-967.) Hybridomas producing monoclonal antibodies directed against CD34 can be obtained from the American Type Tissue Collection. (Rockville, MD). In another embodiment, monoclonal antibodies reactive with endothelial cell surface antigens such as VEGFR-1 and VEGFR-2, CD133, or Tie-2 are used. In the embodiment using genetically-altered cell, antibodies are produced against the genetically engineered gene product using standard techniques in the same manner as described above, and then applied to the blood contacting surface of the medical device following matrix application.

Polyclonal antibodies reactive against endothelial cells isolated from the same species as the one receiving the medical device implant may also be used.

The term "stent" herein means any medical device which when inserted or implanted into the lumen of a vessel expands the cross-sectional lumen of a vessel. The term "stent" includes, but not limited to stainless steel stents, biodegradable stents commercially available which have been coated by the methods of the invention; covered stents such as those covered with PTFE or ePTFE. In one embodiment, this includes stents delivered percutaneously to treat coronary artery occlusions or to seal dissections or aneurysms of the splenic, carotid, iliac and popliteal vessels. In another embodiment, the stent is delivered into a venous vessel. The stent can be composed of polymeric or metallic structural elements onto which the matrix bioerodible, biodegradable, biocompatible polymer comprising the pharmaceutical substance and the ligand such as antibodies is applied, or the stent can be a composite of the matrix intermixed with a polymer. For example, a deformable metal wire stent can be used, such as that disclosed in U.S. Pat. No. 4,886,062 to Wiktor.

Self-expanding stent of resilient polymeric material such as that disclosed in published international patent application WO91/12779 "Intraluminal Drug Eluting Prosthesis", can also be used. Stents may also be manufactured using stainless steel, polymers, nickel-titanium, tantalum, gold, platinum-iridium, or Elgiloy and MP35N and other ferrous materials. Stents are delivered through the body lumen on a catheter to the treatment site where the stent is released from the catheter, allowing the stent to expand into direct contact with the lumenal wall of the vessel. In another embodiment, the stent comprises a biodegradable stent (H. Tamai, pp 297 in Handbook_of Coronary_Stents, 3rd_Edition, Eds. PW Serruys and MJB Kutryk, Martin Dunitz (2000). It will be apparent to those skilled in the art that other self-expanding stent designs (such as resilient metal stent designs) could be used with the antibodies, growth factors and matrices of this invention.

The term "synthetic graft" means any artificial prosthesis having biocompatible characteristics. In one embodiment, the synthetic grafts can be made of polyethylene terephthalate (Dacron®, PET) or polytetrafluoroehtylene (Teflon®, ePTFE). In another embodiment, synthetic grafts are comprised of for example, polyurethane, cross-linked PVA hydrogel, and/or biocompatible foams of hydrogels. In yet a third embodiment, a synthetic graft is composed of an inner layer of meshed polycarbonate urethane and an outer layer of meshed polyethylene terephthalate. It will be apparent to those skilled in the art that any biocompatible synthetic graft can be used with the matrices, pharmaceutical substance and ligands of this invention. (Bos et al. 1998. Small-Diameter Vascular Prostheses: Current Status. Archives Physio Biochem. 106:100-115). Synthetic grafts can be used for end-to-end, end to side, side to end, side to side or intraluminal and in anastomosis of vessels or for bypass of a diseased vessel segments, for example, as abdominal aortic aneurysm devices.

In one embodiment, the matrix may further comprise naturally occurring substances such as collagen, fibronectin, vitronectin, elastin, laminin, heparin, fibrin, cellulose or carbon or synthetic materials. A primary requirement for the matrix is that it be sufficiently elastic and flexible to remain unruptured on the exposed surfaces of the stent or synthetic graft to the surrounding tissue.

In order to coat a medical device such as a stent, the stent may be dipped or sprayed with, for example, a liquid solution of the matrix of moderate viscosity. After each layer is applied, the stent is dried before application of the next layer. In one embodiment, a thin, paint-like matrix coating does not exceed an overall thickness of about 100 microns.

In one embodiment, the stent surface may be first functionalized, followed by the addition of a matrix layer. Thereafter, the antibodies are coupled to the surface of the matrix comprising the drug substance. In this aspect of the invention, the techniques of the stent surface creates chemical groups which are functional. The chemical groups such as amines, are then used to immobilize an intermediate layer of matrix, which serves as support for the ligands such as peptides and antibodies.

In another embodiment, a suitable matrix coating solution is prepared by dissolving 480 milligrams (mg) of a drug carrier, such as poly-D, L-lactid (available as R203 of Boehringer Inc., Ingelheim, Germany) in 3 milliliters (ml) of chloroform under aseptic conditions. In principle, however, any biodegradable (or non-biodegradable) matrix that is blood-and tissue-compatible (biocompatible) and can be dissolved, dispersed or emulsified may be used as the matrix if, after application, it undergoes relatively rapid drying to a self-adhesive lacquer- or paint-like coating on the medical device.

Application of Antibodies as Ligands to the Matrix - Antibodies that promote adherence of progenitor endothelial cells are incorporated into the matrix, either covalently or noncovalently. Antibodies may be incorporated into the matrix layer by mixing the antibodies with the matrix coating solution and then applied the mixture to the surface of the device. In general, antibodies are attached to the surface of the outermost layer of matrix that is applied on the luminal surface of the device, so that the antibodies are projecting on the surface that is in contact with the circulating blood. For example, antibodies and other compounds such as peptides including growth factors can be applied to the surface matrix using standard techniques.

In one embodiment, the antibodies are added to a solution containing the matrix. For example, Fab fragments on anti-CD34 monoclonal antibody are incubated with a solution containing human fibrinogen at a concentration of between 500 and 800 mg/dl. It will be appreciated that the concentration of anti-CD34 Fab fragment will vary and that one of ordinary skill in the art could determine the optimal concentration without undue experimentation. The stent is added to the Fab/fibrin mixture and the fibrin activated by addition of concentrated thrombin (at a concentration of at least 1000U/ml). The resulting polymerized fibrin mixture containing the Fab fragments incorporated directly into the matrix is pressed into a thin film (less than 100 µm) on the surface of the stent or synthetic graft. Virtually any type of antibody or antibody fragment can be incorporated in this manner into a matrix solution prior to coating of a stent or synthetic graft.

For example, in another embodiment, whole antibodies with or without antibody fragments can be covalently coupled to the matrix. In one embodiment, the antibodies and for example peptides such as growth factor(s) are tethered covalently the matrix through the use of hetero- or homobifunctional linker molecules. As used herein the term "tethered" refers to a covalent coupling of the antibody to the matrix by a linker molecule. The use of linker molecules in connection with the present invention typically involves covalently coupling the linker molecules to the matrix after it is adhered to the stent. After covalent coupling to the matrix, the linker molecules provide the matrix with a number of functionally active groups that can be used to covalently couple one or more types of antibody. FIG. 1A provides an illustration of coupling via a cross-linking molecule. An endothelial cell, 1.01, binds to an antibody, 1.03, by a cell surface antigen, 1.02. The antibody is tethered to the matrix, 1.05-1.06, by a cross-linking molecule, 1.04. The matrix, 1.05-1.06, adheres to the stent, 1.07. The linker molecules may be coupled to the matrix directly (i.e., through the carboxyl groups), or through well-known coupling chemistries, such as, esterification, amidation, and acylation. The linker molecule may be a di- or triamine functional compound that is coupled to the matrix through the direct formation of amide bonds, and provides amine-functional groups that are available for reaction with the antibodies. For example, the linker molecule could be a polyamine functional polymer such as polyethyleneimine (PEI), polyallylamine (PALLA) or polyethyleneglycol (PEG). A variety of PEG derivatives, e.g., mPEG-succinimidyl propionate or mPEG-N-hydroxysuccinimide, together with protocols for covalent coupling, are commercially available from Shearwater Corporation, Birmingham, Alabama. (See also, Weiner et al., Influence of a polyethyleneglycol spacer on antigen capture by immobilized antibodies. J. Biochem. Biophys. Methods 45:211-219 (2000)).

It will be appreciated that the selection of the particular coupling agent may depend on the type of antibody used and that such selection may be made without undue experimentation. Mixtures of these polymers can also be used. These molecules contain a plurality of pendant amine-functional groups that can be used to surface-immobilize one or more antibodies.

Small molecules can comprise synthetic or naturally occurring molecules or peptides which can be used in place of antibodies or fragments thereof, or in combination with antibodies or antibody fragments. For example, lectin is a sugar-binding peptide of non-immune origin which occurs naturally. The endothelial cell specific Lectin antigen (Ulex Europaeus Uea 1) (Schatz et al. 2000 Human Endometrial Endothelial Cells: Isolation, Characterization, and Inflammatory-Mediated Expression of Tissue Factor and Type 1 Plasminogen Activator Inhibitor. Bioi Reprod 62: 691-697) can selectively bind the cell surface of progenitor endothelial cells.

Synthetic "small molecules" have been created to target various cell surface receptors. These molecules selectively bind a specific receptor(s) and can target specific cell types such as progenitor endothelial cells. Small molecules can be synthesized to recognize endothelial cell surface markers such as VEGF. For example, SU11248 (Sugen Inc.) (Mendel et al. 2003 In vivo antitumor activity of SU11248, a novel tyrosine kinase inhibitor targeting vascular endothelial growth factor and platelet-derived growth factor receptors: determination of a pharmacokinetic/pharmacodynamic relationship. Clin Cancer Res. Jan;9(1):327-37), PTK787/ZK222584 (Drevs J. et al. 2003 Receptor tyrosine kinases: the main targets for new anticancer therapy. Curr Drug Targets. Feb;4(2):113-21) and SU6668 (Laird, AD et al. 2002 SU6668 inhibits Flk-1/KDR and PDGFRbeta in vivo, resulting in rapid apoptosis of tumor vasculature and tumor regression in mice. FASEB J. May;16(7):681-90) are small molecules which bind to VEGFR-2.

Another subset of synthetic small molecules which target the endothelial cell surface are, for example, the alpha(v)beta(3) integrin inhibitors, SM256 and SD983

(Kerr JS. et al. 1999 Novel small molecule alpha v integrin antagonists: comparative anti-cancer efficacy with known angiogenesis inhibitors. Anticancer Res Mar-Apr;19(2A):959-68). SM256 and SD983 are both synthetic molecules which target and bind to alpha(v)beta(3) present on the surface of endothelial cells.

The invention also relates to a method of treating a patient having vascular disease, such as artherosclerosis, and in need of such treatment with the coated medical device of the invention. The method comprises implanting into a patient in need of the treatment a coated medical device of the invention. The methods of the invention may be practiced *in vivo* or *in vitro.*

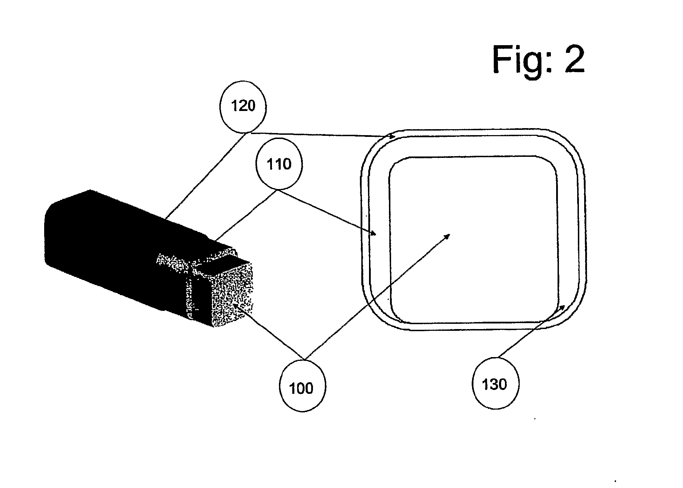

The coating of the invention can be applied using various techniques available in the art, such as dipping, spraying, vapor deposition, injection like and/or dot matrix-like approach. For example, FIG. 1 illustrates a simple pattern of cell capturing and drug delivery mechanism in which a stent strut 100 is shown with a continuous coating of a drug/polymer matrix layer 110 applied to the strut surface and a ligand layer 120 on top of the drug/polymer composition. FIG. 2 illustrates an alternate embodiment of the invention in which the drug/polymer layer 110 is a discontinuous layer 130, however, the amount of drug/polymer matrix composition greater than the, for example, that shown in FIG 2.

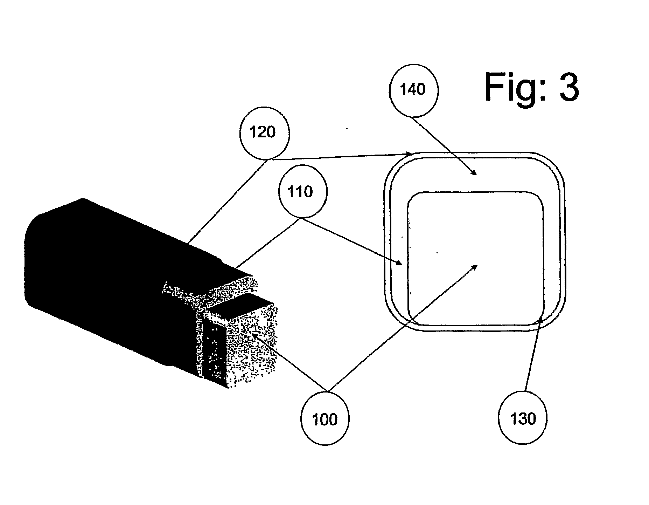

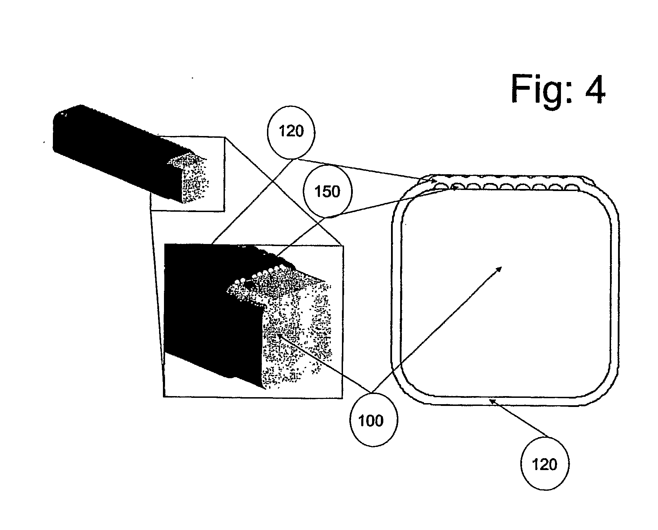

FIG. 3 shows an alternate embodiment in which the drug/polymer layer is discontinuous. In this embodiment, the drug/polymer composition is applied to about % of the circumference of the device, however, the middle one third 140 of the layer 110 comprises the greatest amount of the drug composition, and the ligand layer is applied on top of drug/polymer layer. FIG. 4 shows yet another embodiment with respect to the application of the coating. In this embodiment of the invention, the drug/polymer matrix composition is applied to a portion of the surface of the medical device 100 in a dot matrix like pattern 150. As seen in FIG. 4, the ligand layer 120 is applied to surrounds the entire circumference of the medical device including the drug/polymer composition 110.

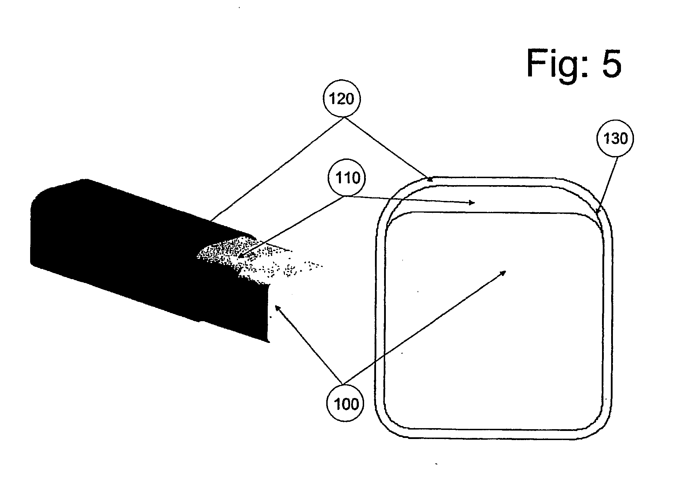

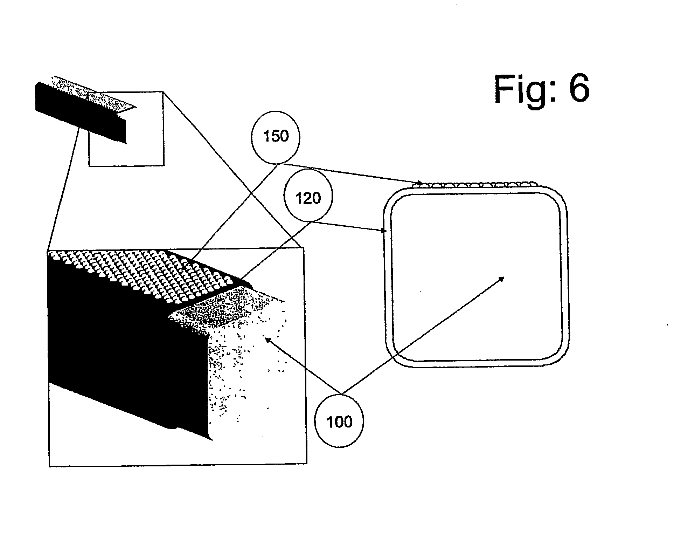

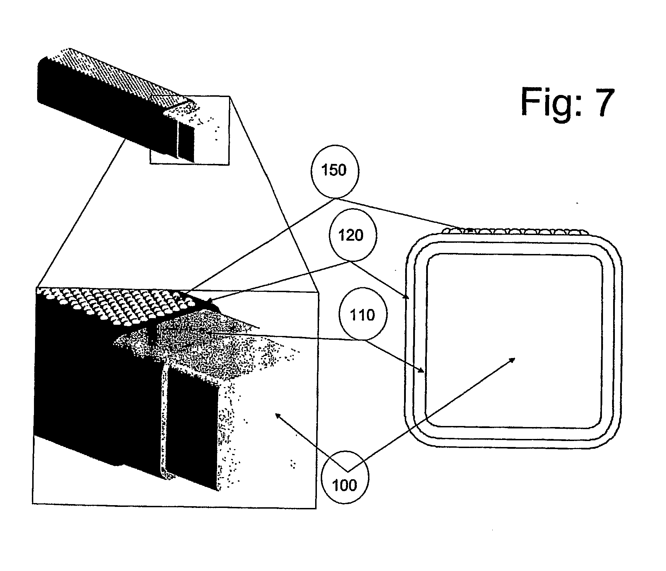

In yet another embodiment, FIG. 5 shows a medical device 100 coated with a drug/polymer matrix composition which is concentrated in a small section of the surface 110 of the device 100. In this aspect of the invention, the ligand layer 120 covers the entire circumference of the device including the drug/polymer composition 110. FIG. 6 shows an alternate embodiment in which the ligand layer 120 is applied to cover the surface of device 100 and in a section of the surface of ligand layer 120, a drug/polymer matrix composition 150 is applied on the device. FIG. 7 shows an alternate embodiment, in which the device can be covered with multiple layers of drug/polymer matrix composition 110, 150 applied as a continuous layer 110 on the surface of the device 100, followed by a ligand layer 120 and an additional drug/polymer matrix discontinuous layer in a dot matrix like patter 150 on the surface of the ligand layer 120.

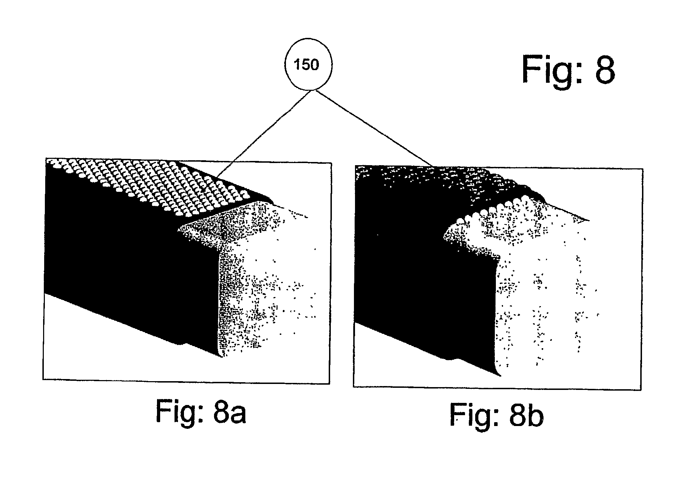

Additional alternate embodiments are shown in FIGs. 8A and 8B. In this aspect of the invention, the medical device, in this case a stent strut is coated with a ligand layer 120 and a drug/polymer matrix layer in a dot matrix pattern 150 can be partially applied on device on top of the ligand layer (FIG. 8A) or below (FIG. 8B) the ligand layer.

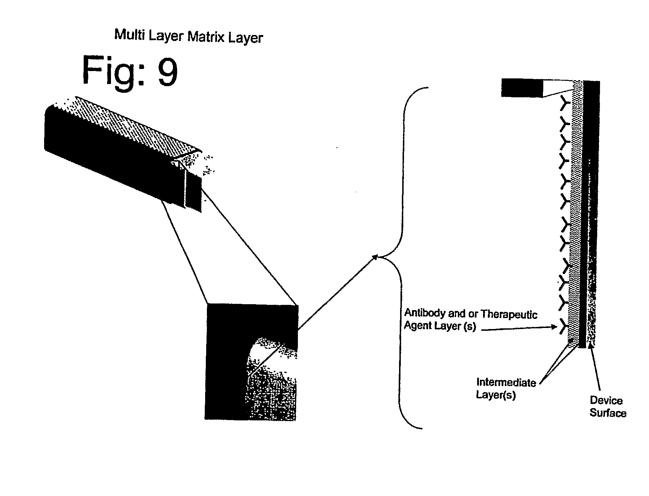

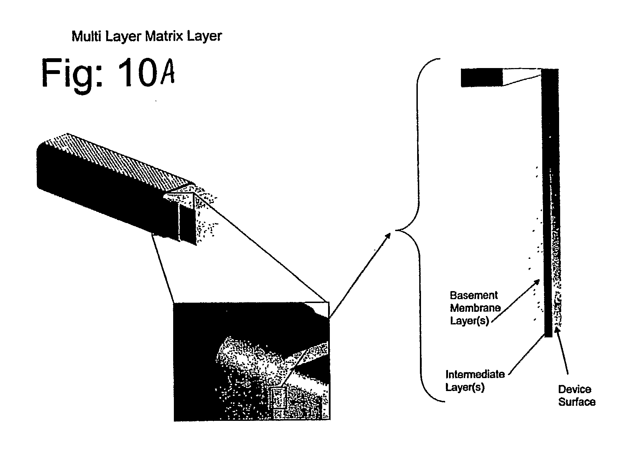

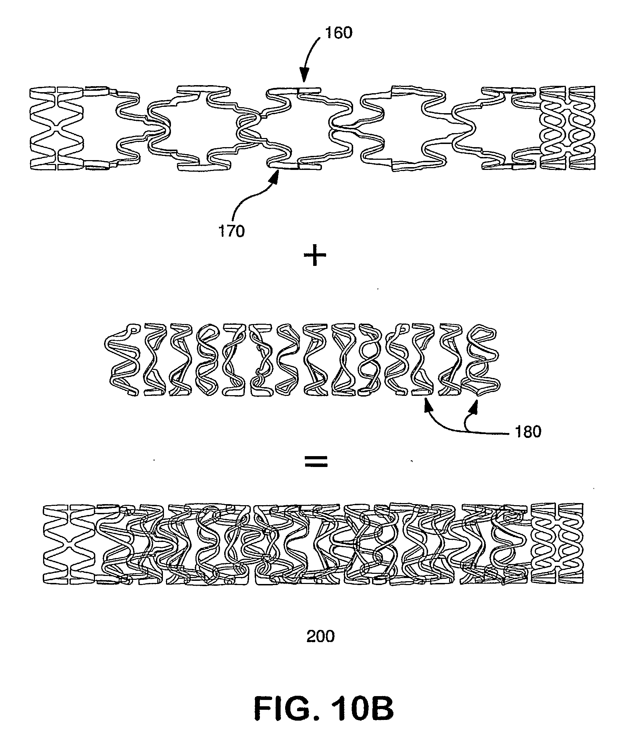

FIGs. 9 and 10 show other embodiments of the invention in cross-section. As seen in FIG. 9, the ligand, such as an antibody is shown as the outermost layer on the surface of the coated medical device, and the coating can comprise additional intermediate layers, which comprise the drug/polymer composition and optionally additional components. FIG. 10A additionally illustrates a basement membrane and an intermediate layer coating the device.

In another embodiment comprising a stent, the coating composition comprising a drug/polymer matrix, can be applied to portions of the stent such as the spine or helical element of a stent. In this aspect of the invention, the remaining surfaces of the stent not covered with the drug/polymer matrix can be coated with the ligand layer on portions of the stent surface or the entire remaining surface of the stent as illustrated in FIG. 10B. In the embodiment in FIG. 10B, the pharmaceutical release component and the antibody modified surface are exposed on alternating surfaces of the device. This allows for more targeted treatment of segments of the vessel (such as the healthier tissue at the leading and trailing ends of the stent versus the highly diseased middle portion of the stent, i.e., center of the lesion) and minimizes the interaction between the pharmaceutical component the antibody surface, and the newly adhered endothelial cells on the surface of the stent.

As illustrated in FIG. 10B, the stent ends component may be comprised of for example, an antibody or a small molecule (EPC capture) surface. Helix component 160 can comprised of a basement membrane base coating, and helix segment 170 represents a slow release pharmaceutical compontent that can be comprised of a non-degradeable biocompatible polymer matrix that elutes an agent for maintaining long term vessel patency such as eNOS, tPA, statins, and/or antiboitics. FIG. 10B also shows the ring component 180 of the stent can be comprised of a fast release pharmaceutical agent to retard early neointimal hyperplasia/smooth muscle cell migration, and the entire stent 200 is therefore coated with different coating in each portion of the device.

The following examples illustrate the invention, but in no way limit the scope of the invention.

### Reference example 1

### Preparation of coating composition

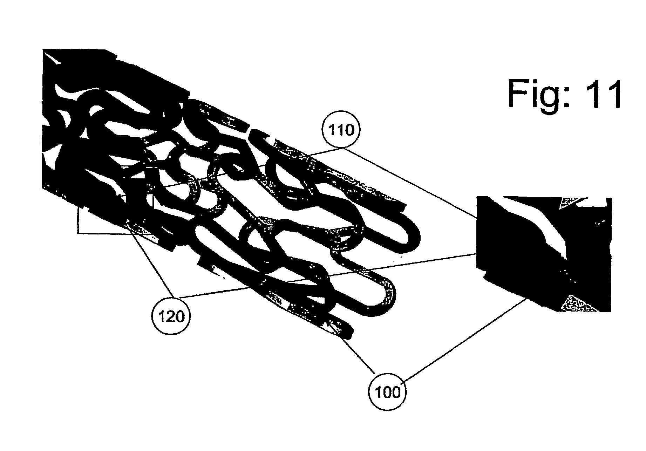

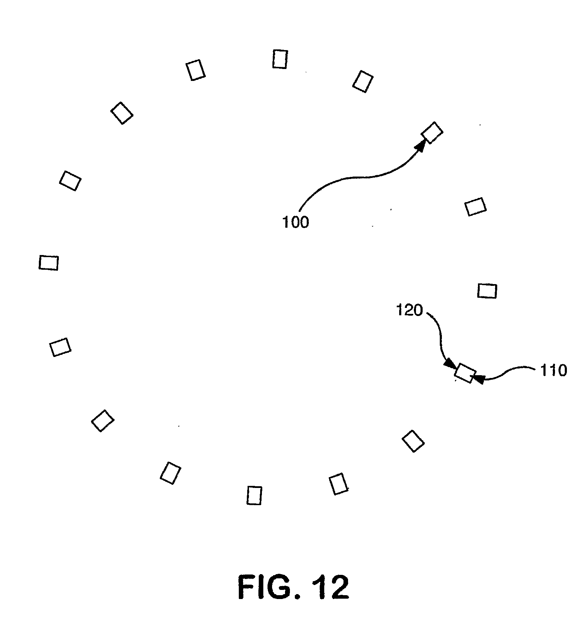

The polymer Poly DL Lactide-co-Glycolide (DLPLG, Birmingham Polymers) is provided as a pellet. To prepare the polymer matrix composition for coating a stent, the pellets are weighed and dissolved in a ketone or methylene chloride solvent to form a solution. The drug is dissolved in the same solvent and added to the polymer solution to the required concentration, thus forming a homogeneous coating solution. To improve the malleability and change the release kinetics of the coating matrix, the ratio of lactide to glycolide can be varied. This solution is then used to coat the stent to form a uniform coating as shown in FIG. 11. FIG. 12 shows a cross-section through a coated stent of the invention. The polymer(s)/drug(s) composition can be deposited on the surface of the stent using various standard methods.

### Reference example 2

### Evaluation of Polymer/Drugs and Concentrations

Process for Spray-Coating Stents: The polymer pellets of DLPLG which have been dissolved in a solvent are mixed with one or more drugs. Alternatively, one or more polymers can be dissolved with a solvent and one or more drugs can be added and mixed. The resultant mixture is applied to the stent uniformly using standard methods. After coating and drying, the stents are evaluated. The following list illustrates various examples of coating combinations, which were studied using various drugs and comprising DLPLG and/or combinations thereof. In addition, the formulation can consist of a base coat of DLPLG and a top coat of DLPLG or another polymer such as DLPLA or EVAC 25. The abbreviations of the drugs and polymers used in the coatings are as follows: MPA is mycophenolic acid, RA is retinoic acid; CSA is cyclosporine A; LOV is Iovastatin.TM. (mevinolin); PCT is Paclitaxel; PBMA is Poly butyl methacrylate, EVAC is ethylene vinyl acetate copolymer; DLPLA is Poly (DL Lactide), DLPLG is Poly(DL Lactide-co-Glycolide).

Examples of the coating components and amounts (%) which can be used in the invention comprise:
1. 50% MPA/50% Poly L Lactide
2. 50% MPA/50% Poly DL Lactide
3. 50% MPA/50% (86:14 Poly DL Lactide-co-Caprolactone)
4. 50% MPA/50% (85:15 Poly DL Lactide-co-Glycolide)
5. 16% PCT/84% Poly DL Lacide
6. 8% PCT/92% Poly DL Lactide
7. 4% PCT/92% Poly DL Lactide
8. 2% PCT/92% Poly DL Lactide
9. 8% PCT/92% of (80:20 Poly DL Lactide/EVAC 40)
10. 8% PCT/92% of (80:20 Poly DL Lactide/EVAC 25)
11. 4% PCT/96% of (50:50 Poly DL Lactide/EVAC 25)
12. 8% PCT/92% of (85:15 Poly DL Lactide-co-Glycolide)
13. 4% PCT/96% of (50:50 Poly DL Lactide-co-Glycolide)
14. 25% LOV/25% MPA/50% of (EVAC 40/PBMA)
15. 50% MPA/50% of (EVAC 40/PBMA)
16. 8% PCT/92% of (EVAC 40/PBMA)
17. 8% PCT/92% EVAC 40
18. 8% PCT/92% EVAC 12
19. 16% PCT/84% PBMA
20. 50% CSA/50% PBMA
21. 32% CSA/68% PBMA
22. 16% CSA/84% PBMA

### Reference example 3

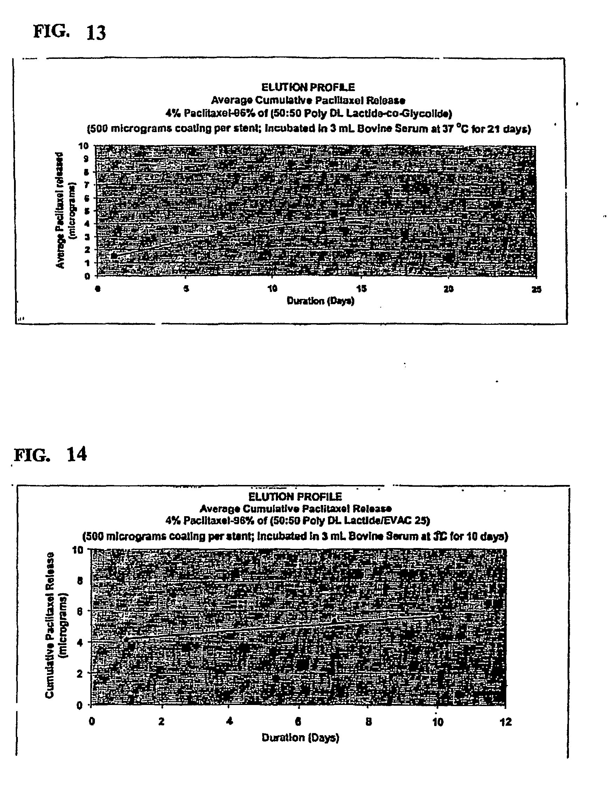

The following experiments were conducted to measure the drug elution profile of the coating on stents coated by the method described in Reference example 2. The coating on the stent consisted of 4% Paclitaxel and 96% of a 50:50 Poly(DL-Lactide-co-Glycolide) polymer. Each stent was coated with 500 .mu.g of coating composition and incubated in 3 ml of bovine serum at 37.degree. C. for 21 days. Paclitaxel released into the serum was measured using standard techniques at various days during the incubation period. The results of the experiments are shown in FIG. 13. As shown in FIG. 13, the elution profile of Paclitaxel release is very slow and controlled since only about 4 µg of Paclitaxel are released from the stent in the 21-day period.

### Reference example 4

The following experiments were conducted to measure the drug elution profile of the coating on stents coated by the method describe in Reference example 2. The coating on the stent consisted of 4% Paclitaxel and 92% of a 50:50 of Poly(DL-Lactide) and EVAC 25 polymer. Each stent was coated with 500 µg of coating composition and incubated in 3 ml of bovine serum at 37°C for 10 days. Paclitaxel released into the serum was measured using standard techniques at various days during the incubation period. The results of the experiments are shown in FIG. 14. As shown in FIG. 14, the elution profile of Paclitaxel release is very slow and controlled since only about 6 µg of Paclitaxel are released from the stent in the 10-day period.

### Reference example 5

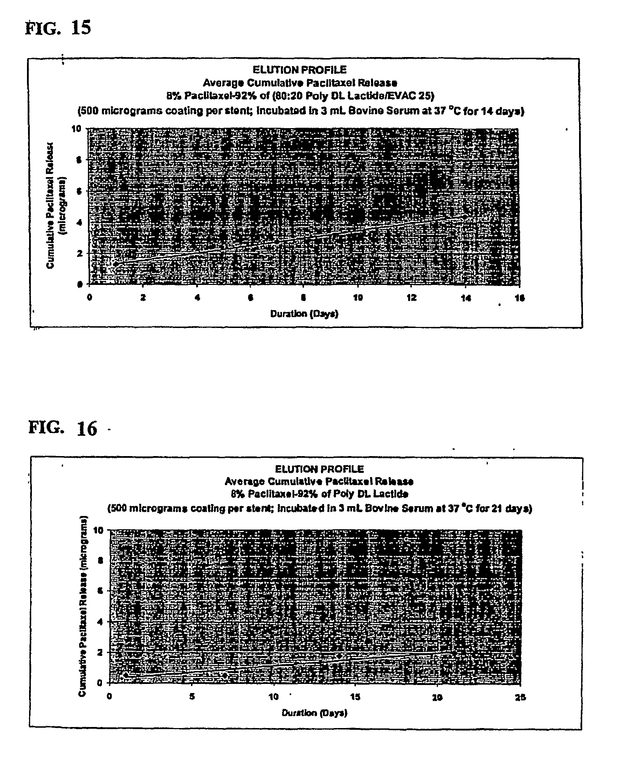

[0077] The following experiments were conducted to measure the drug elution profile of the coating on stents coated by the method describe in Reference example 2. The coating on the stent consisted of 8% Paclitaxel and 92% of a 80:20 of Poly(DL-Lactide) and EVAC 25 polymer. Each stent was coated with 500 µg of coating composition and incubated in 3 ml of bovine serum at 37°C. for 14 days. Paclitaxel released into the serum was measured using standard techniques at various days during the incubation period. The results of the experiments are shown in FIG. 15. As shown in FIG. 15, the elution profile of Paclitaxel release is very slow and controlled since only about 4 µg of Paclitaxel are released from the stent in the 14-day period.

### Reference example 6

The following experiments were conducted to measure the drug elution profile of the coating on stents coated by the method describe in Reference example 2. The coating on the stent consisted of 8% Paclitaxel and 92% of Poly(DL-Lactide) polymer. Each stent was coated with 500 µg of coating composition and incubated in 3 ml of bovine serum at 37°C for 21 days. Paclitaxel released into the serum was measured using standard techniques at various days during the incubation period. The results of the experiments are shown in FIG. 16. As shown in FIG. 16, the elution profile of Paclitaxel release is very slow and controlled since only about 2 µg of Paclitaxel are released from the stent in the 21-day period. The above data show that by varying the polymer components of the coating, the release of a drug can be controlled for a period of time required.

### Reference example 7

In this experiments, the elution profile of stents coated with a composition comprising 92% PGLA and 9% paclitaxel as described in Reference example 2 were measured. Elution testing is used to provide data for the release kinetics of the paclitaxel from the polymer matrix. The release of the paclitaxel into bovine calf serum at 37°C was used to approximate the *in vivo* conditions. While serum is similar to blood, this simulation does not necessarily reflect the actual release kinetics of the implanted device. This simulation provides a repeatable, controlled environment from which relative release may be evaluated. Elution data is collected on sets of paclitaxel coated stents comprised of 0.13, 0.20, 0.29, 0.38 µg/mm² paclitaxel. The 0.13 and 0.26 µg/mm² units were evaluated in animal testing studies.

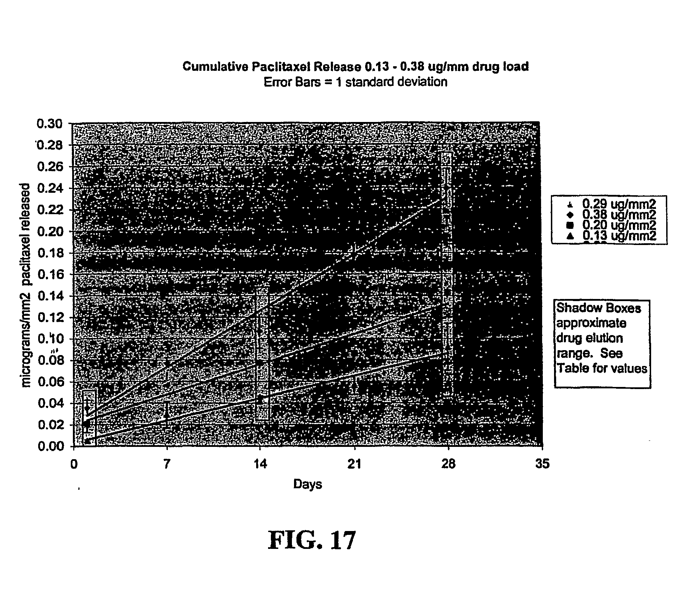

**Elution Test method:** Coated stents are placed in bovine calf serum at 37°C. At designated time points, the stents are removed from the serum. The residual paclitaxel is extracted from the coating. The amount of paclitaxel released is calculated by subtracting the amount of paclitaxel remaining on the stent from the original loaded amount of paclitaxel loaded onto the stent. FIG. 17 demonstrates the amount of paclitaxel released per square millimeter of stent surface. Table 1 shows the range of *in vitro* release kinetics at 1, 14 and 28 days. As seen in FIG. 16 and Table 1, the release kinetics of the coating is slow as the paclitaxel ranges from 0 to 0.051 µg/mm² on Day 1 to 0.046 to 0.272 µg/mm² on Day 28.

**Table 1**

| | **1 Day Micrograms/mm²** | **14 Days Micrograms/mm²** | **28 Days Micrograms/mm²** |
|---|---|---|---|
| **Average** | 0.021 | 0.087 | 0.158 |
| **Maximum** | 0.051 | 0.148 | 0.272 |
| **Minimum** | 0.00 | 0.023 | 0.046 |

### Reference example 8

Additional serum elution data were performed out to 70 days and 48 days with stents coated with 4% Paclitaxel/96% PGLA and 100% PGLA respectively. The elution of paclitaxel is monitored by analyzing the amount of paclitaxel in the serum out to 42 days as reported. A test method which monitors the amount of residual paclitaxel on the stent is used to characterize the elution at 90 days for TG0331A. The residual paclitaxel on 5 stents available for testing gave an average of 2.29 micrograms (range 1.87 - 2.86) maximum.

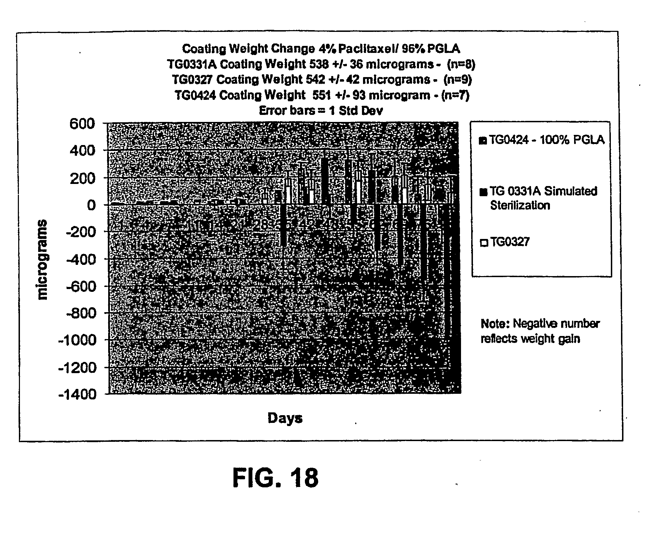

The weight of the coated stents was measured at specified time points during the elution in serum at 37°C. Comparison of non-treated and simulated sterilization units (40°C, 18 hours) demonstrates a difference in the weight loss profile. Also the weight loss of PGLA without drug is shown for comparison. FIG. 18 shows the results of these experiments. As seen in FIG. 18, simulated sterilization causes a gain in weight of the coated stents.

At each time point during the experiments, the stent coatings are microscopically examined and photographs. Table 2 below shows some visual characteristics of the Samples #1-3.

**Table 2**

| **Sample No.** | **Description** | **Time points** | **Observation *** |
|---|---|---|---|
| 1 | 4% Paclitaxel Simulated Sterilization | 63 Days | Coating no longer has smooth appearance and some areas where no coating present |
| | | 70 Days | Similar to 63 days, with more coating missing, but not as much missing as 78 days for TG0327 |
| | | 84 Days | Similar to sample #3 at 48 and 62 days |
| 2 | 4% Paclitaxel (no sim sterile) | 21 Days | Smooth coating, white appearance, some bubbles on surface |
| | | 28 Days | Coating no longer smooth, some coating missing |
| | | 78 Days | Similar to TG0331A with more coating missing |
| | | 90 Days | Similar to sample #3 at 62 Days. |
| 3 | 100% PGLA | 48 Days | Coating not smooth and some coating missing |
| | | 62 Days | Significant areas of stent with coating missing. |
| | | 90 Days | Small amounts of remaining coating. |

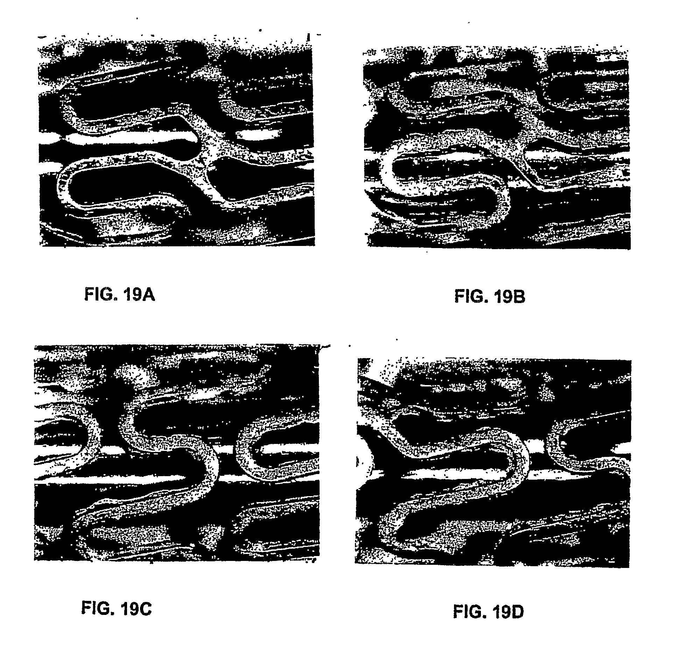

FIG 19A-19D shows that virtually all the drug present in the coating has eluted after 90 days of serum incubation, while some polymer matrix remains attached to the stent. The combination of weight change, drug elution, and microscopic evaluation provides a good characterization of the coated surface. Both Samples #2 and #3 did not see the simulated sterilization condition and responded more similarly. The samples subjected to simulated sterilization conditions, Sample #1 appears to have a slower degradation rate of the coating in serum. A trend is seen in the coating appearance under microscope that the amount of coating remaining for this group. This makes sense as the simulated sterilization conditions is just below the Tg of the polymer and may cause some annealing of the material.

The drug elution at 90 days demonstrates that virtually all the drug has been eluted from the coating. The amount of drug measured is a maximum as degraded polymer will also result in some absorbance at the test wavelength. Considering testing on other lots for residual drug demonstrated roughly 80% of the drug is eluted after 28 days in serum.

These results provide evidence that the polymer is still present but that the drug is substantially eluted at 90 days from a 4% paclitaxel loaded PGLA matrix in serum.

### Reference example 9

**Endothelial Cell Capture by anti-CD34 coated Stainless Steel Disks:** Human Umbilical Vein Endothelial Cells (HUVEC) (American Type Culture Collection) are grown in endothelial cell growth medium for the duration of the experiments. Cells are incubated with CMDX and gelatin coated samples with or without bound antibody on their surface or bare stainless steel (SST) samples. After incubation, the growth medium is removed and the samples are washed twice in PBS. Cells are fixed in 2% paraformaldehyde (PFA) for 10 minutes and washed three times, 10 minutes each wash, in PBS, to ensure all the fixing agent is removed. Each sample is incubated with blocking solution for 30 minutes at room temperature, to block all non-specific binding. The samples are washed once with PBS and the exposed to 1:100 dilution of VEGFR-2 antibody and incubated overnight. The samples are subsequently washed three times with PBS to ensure all primary antibody has been removed. FITC-conjugated secondary antibody in blocking solution is added to each respective sample at a dilution of 1:100 and incubated for 45 minutes at room temperature on a Belly Dancer apparatus. After incubation, the samples are washed three times in PBS, once with PBS containing 0.1 % Tween 20, and then again in PBS. The samples are mounted with Propidium Iodine (PI) and visualized under confocal microscopy.

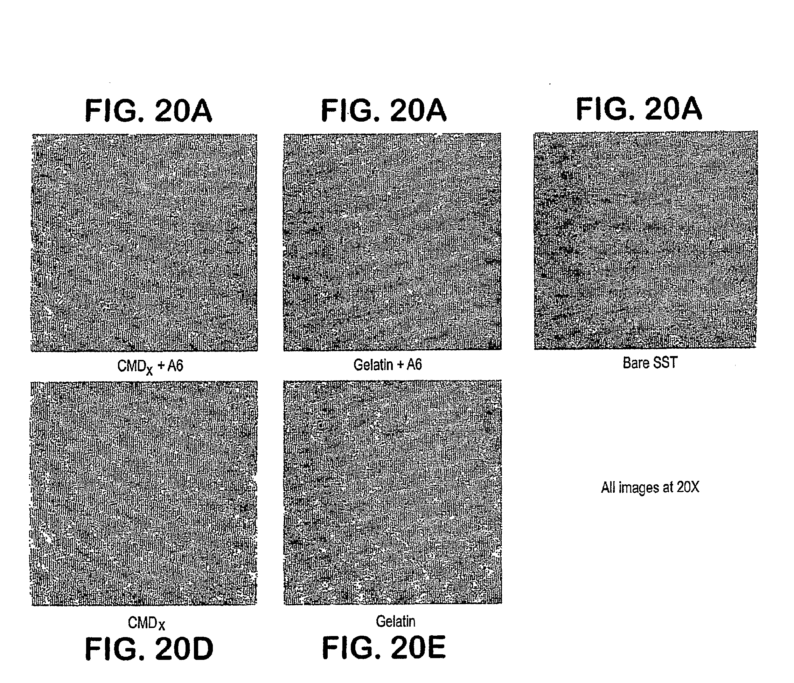

FIGs. 20A-4E are photomicrographs of SST samples coated with CMDX and anti-CD34 antibody (FIG. 20A), gelatin and anti-CD34 antibody coated (FIG. 20B), bare SST (FIG. 20C), CMDX coated and no antibody (FIG, 20D) and gelatin-coated and no antibody (FIG. 20E). The figures show that only the antibody coated samples contain numerous cells attached to the surface of the sample as shown by PI staining. The bare SST control disk shows few cells attached to its surface.

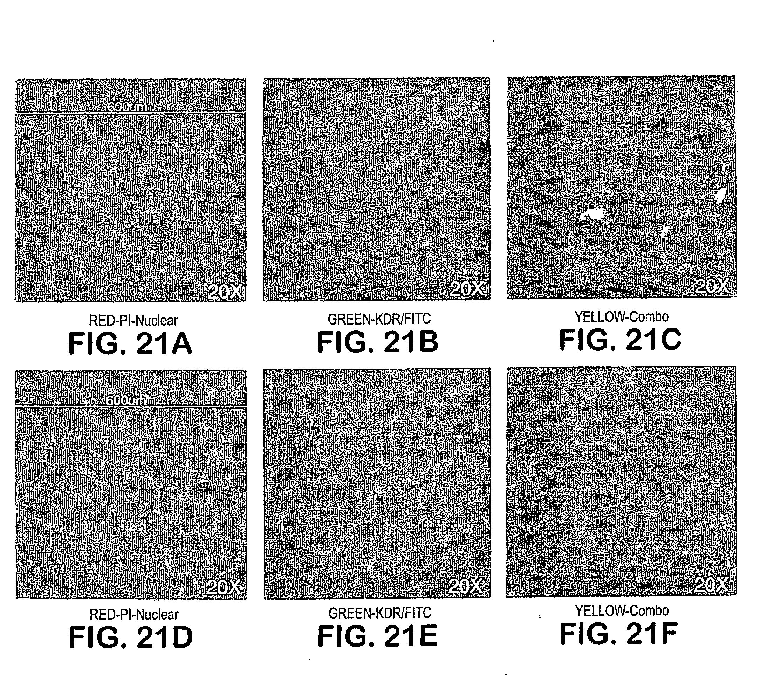

FIGs. 21A-21C are photomicrographs of control samples CMDX-coated without antibody bound to its surface. FIG. 21A shows very few cells as seen by PI staining adhered to the surface of the sample. FIG. 21B shows that the adherent cells are VEGFR-2 positive indicating that they are endothelial cells and FIG. 21C shows a combination of the stained nuclei and the VEGFR-2 positive green fluorescence. FIGs. 21D-F are photomicrographs of control samples coated with gelatin without antibody on its surface. FIG. 21D shows no cells are present since PI staining is not present in the sample and there is no green fluorescence emitted by the samples (see FIGs. 21E and 21F).

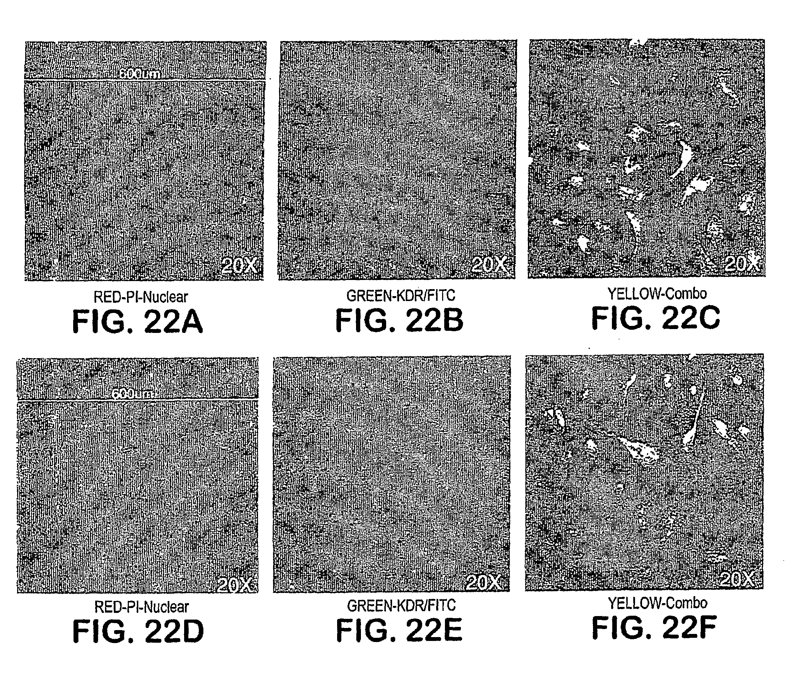

FIGs. 22A-22C are photomicrographs of CMDX coated SST samples having anti-CD34 antibody bound on its surface. The figures show that the samples contain numerous adherent cells which have established a near confluent monolayer (FIG. 22A) and which are VEGFR-2 positive (FIGs. 22B and 22C) as shown by the green fluorescence. Similarly, FIGs. 22D-22F are photomicrographs of a gelatin-coated sample with anti-CD34 antibody bound to its surface. These figures also show that HUVECs attached to the surface of the sample as shown by the numerous red-stained nuclei and green fluorescence from the VEGFR-2/FITC antibody (FIGs. 22E and 22F).

## Claims

1. A medical device comprising a blood contacting or luminal surface and a coating for controlled release of one or more pharmaceutical substances to adjacent tissue, comprising one or more layers of a bio-absorbable or non-absorbable, biocompatible matrix, comprising a pharmaceutical substance that inhibits smooth muscle cell migration or proliferation, and one or more ligands selected from an antibody, an antibody fragment, and combinations thereof, said ligands being covalently attached to said matrix and configured to bind *in situ* to cell surface antigens or cell membrane molecules of circulating progenitor endothelial cells in peripheral blood for *in vivo* capture and proliferation on the blood contacting surface of the medical device.

2. The medical device of claim 1, wherein the device is structured and configured to be implanted in a patient, and wherein at least one surface of the device comprises one or more base materials.

3. The medical device of claim 1, wherein the medical device is a stent, a vascular or other synthetic graft, or a stent in combination with a synthetic graft.

4. The medical device of claim 1, wherein the medical device is a vascular stent.

5. The vascular stent of claim 4, wherein the stent structure comprises a biodegradable material.

6. The vascular stent of claim 5. wherein the biodegradable material gradually releases said pharmaceutical substance that inhibits smooth muscle cell migration or proliferation into adjacent vascular tissue.

7. The medical device of claim 2, wherein the base material is biocompatible.

8. The medical device of claim 2, wherein the base material is selected from the group consisting of stainless steel, Nitinol, MP35N. gold, tantalum, platinum or platinum iridium, biocompatible metals and/or alloys, carbon fiber, cellulose acetate.
cellulose nitrate, silicone, cross-linked polyvinyl acetate (PVA) hydrogel, cross-linked PVA hydrogel foam, polyurethane, polyamide, styrene isobutylene-styrene block copolymer (Kraton), polyethylene terephthalate, polyurethane, polyamide, polyester, polyorthoester, polyanhydride, polyether sulfone, polycarbonate, polypropylene, high molecular weight polyethylene, polytetrafluoroethylene, polyesters of polylactic acid, polyglycolic acid, copolymers thereof, a polyanhydride, polycaprolactone, polyhydroxybutyrate valerate, extracellular matnx components, proteins, elastin, collagen, fibrin, and mixtures thereof

9. The medical device of claim 1. wherein the bioabsorbable matrix comprises one or more polymers or oligomers selected from the group consisting of poly(lactide-co-glycolide), polylactic acid, polyglycolic acid, a polyanhydride, polycaprolactone, polyhydroxybutyrate valerate, copolymers thereof and combinations thereof.

10. The medical device of claim 1, wherein the coating comprises poly(DL-lactide-co-glycolide) and one or more pharmaceutical substances.

11. The medical device of claim 9, wherein the bio-absorbable matrix comprises poly(DL-lactide).

12. The medical device of claim 9, wherein the bio-absorbable matrix comprises poly(DL-lactide) or poly(lactide-co-glycolide) and the pharmaceutical substance is paclitaxel.

13. The medical device of any of claims 1 to 11, wherein the pharmaceutical substance is selected from the group consisting of cyclosporin A, mycophenolic acid, mycophenolate mofetil acid, rapamycin, rapamycin derivatives, azathioprene, tacrolimus, tranilast, dexamethasone, corticosteroid, everolimus, retinoic acid, vitamin E, rosglitazone, simvastatins, fluvastatin, estrogen, 17β-estradiol, dihydroepiandrosterone, testosterone, puerarin, platelet factor 4, basic fibroblast growth factor, fibronectin, butyric acid, butyric acid denvatives, paclitaxel, paclitaxel derivatives and probucol.

14. The medical device of claim 13, wherein the pharmaceutical substances comprise rapamycin, rapamycin derivatives, paclitaxel, paclitaxel derivatives and sirolimus.

15. The medical device of any of claims 1 to 13, wherein the pharmaceutical substances are cyclosporin A and mycophenolic acid.

16. The medical device of any of claims 1 to 13, wherein the pharmaceutical substances are mycophenolic acid and vitamin E.

17. The medical device of any of claims 1 to 16, wherein the pharmaceutical substance comprises from about 1 to about 50% (w/w) of the composition.

18. The medical device of claim 10, wherein the poly(DL-lactide) polymer comprises from about 50 to about 99% of the composition.

19. The medical device of any of claims 1 to 18, further comprising a nonabsorbable polymer.

20. The medical device of claim 19, wherein the nonabsorbable polymer is methylmethacrylate.

21. The medical device of claim 1, wherein the coating comprises a single homogeneous layer of poly(DL-lactide) polymer or poly(lactide-co-glycolide) and the pharmaceutical substances.

22. The medical device of claim 1, wherein the coating comprises multiple layers of poly(DL-lactide) polymer or poly(lactide-co-glycolide) copolymer, or a mixture thereof.

23. The medical device of claim 1, wherein the coating comprises multiple layers of the pharmaceutical substances.

24. The medical device of any of claims 1 to 23, wherein the ligand is attached to the blood contacting surface of the medical device.

25. The medical device of any of claims 1 to 24, wherein the ligand is an antibody or a peptide which hinds to a progenitor cell surface antigen.

26. The medical device of claim 1, wherein the polymer matrix is poly(DL-lactide-co-glycolide) in the ratio of 50:50 having a molecular weight of 75,000 to 100,000.

27. A method for coating the medical device of claim 1, comprising:
applying to the surface of said medical device at least one layer of a composition comprising a controlled-release polymer matrix, at least one pharmaceutical substance, and optionally a basement membrane component;
applying to said at least one layer of said composition on said medical device a solution comprising at least one type of ligand for binding and immobilizing progenitor endothelial cells; and
drying said coating on the medical device under vacuum at low temperatures.

## Patentansprüche

1. Medizinische Vorrichtung, umfassend eine mit Blut in Kontakt kommende oder luminale Oberfläche und eine Beschichtung zur kontrollierten Abgabe von einer oder mehreren pharmazeutischen Substanzen an angrenzendes Gewebe, die eine oder mehrere Schichten aus einer bioabsorbierbaren oder nicht absorbierbaren biokompatiblen Matrix aufweist, die Folgendes umfasst:
eine pharmazeutische Substanz, welche die Migration oder Proliferation von glatten Muskelzellen hemmt, und einen oder mehrere Liganden, die aus einem Antikörper, einem Antikörperfragment und Kombinationen davon ausgewählt sind, wobei die Liganden kovalent an die Matrix gebunden sind und dafür ausgelegt sind, *in situ* an Zelloberflächenantigene oder Zellmembranmoleküle von zirkulierenden endothelialen Vorläuferzellen in peripherem Blut zu binden,
damit diese *in vivo* an der mit Blut in Kontakt kommenden Oberfläche der medizinischen Vorrichtung erfasst werden und dort proliferieren.

2. Medizinische Vorrichtung nach Anspruch 1, wobei die Vorrichtung so strukturiert und aufgebaut ist, dass sie in einen Patienten implantiert werden kann, und wobei wenigstens eine Oberfläche der Vorrichtung ein oder mehrere Grundmaterialien umfasst.

3. Medizinische Vorrichtung nach Anspruch 1, wobei die medizinische Vorrichtung ein Stent, ein Gefäßtransplantat oder ein anderes synthetisches Transplantat oder ein Stent in Kombination mit einem synthetischen Transplantat ist.

4. Medizinische Vorrichtung nach Anspruch 1, wobei die medizinische Vorrichtung ein Gefäßstent ist.

5. Gefäßstent nach Anspruch 4, wobei die Stentstruktur ein biologisch abbaubares Material umfasst.

6. Medizinische Vorrichtung nach Anspruch 5, wobei das biologisch abbaubare Material die pharmazeutische Substanz, welche die Migration oder Proliferation von glatten Muskelzellen hemmt, allmählich an das angrenzende Gefäßgewebe abgibt.

7. Medizinische Vorrichtung nach Anspruch 2, wobei das Grundmaterial biokompatibel ist.

8. Medizinische Vorrichtung nach Anspruch 2, wobei das Grundmaterial aus der Gruppe ausgewählt ist, die aus rostfreiem Stahl, Nitinol, MP35N, Gold, Tantal, Platin oder Platin/Irdium, biokompatiblen Metallen und/oder Legierungen, Kohlenstofffasern, Celluloseacetat, Cellulosenitrat, Silikon, Hydrogel aus vernetztem Polyvinylacetat (PVA), Hydrogel-Schaum aus vernetztem PVA, Polyurethan, Polyamid, Styrol-Isobutylen-Styrol-Blockcopolymer (Kraton), Polyethylenteraphthalat, Polyurethan, Polyamid, Polyester, Polyorthoester, Polyanhydrid, Polyethersulfon, Polycarbonat, Polypropylen, Polyethylen mit hohem Molekulargewicht, Polytetrafluorethylen, Polyester von Polymilchsäure, Polyglycolsäure, Copolymeren davon, einem Polyanhydrid, Polycaprolacton, Polyhydroxybutyrat-Valerat, extrazellulären Matrixkomponenten, Proteinen, Elastin, Collagen, Fibrin und Mischungen davon besteht.

9. Medizinische Vorrichtung nach Anspruch 1, wobei die bioabsorbierbare Matrix ein oder mehrere Polymere oder Oligomere umfasst, die aus der Gruppe ausgewählt sind, die aus Poly(lactid-co-glycolid), Polymilchsäure, Polyglycolsäure, einem Polyanhydrid, Polycaprolacton, Polyhydroxybutyrat-Valerat, Copolymeren davon und Kombinationen davon besteht.

10. Medizinische Vorrichtung nach Anspruch 1, wobei die Beschichtung Poly(DL-lactid-co-glycolid) und eine oder mehrere pharmazeutische Substanzen umfasst.

11. Medizinische Vorrichtung nach Anspruch 9, wobei die bioabsorbierbare Matrix Poly(DL-lactid) umfasst.

12. Medizinische Vorrichtung nach Anspruch 9, wobei die bioabsorbierbare Matrix Poly(DL-lactid), Poly(lactid-co-glycolid) umfasst und die pharmazeutische Substanz Paclitaxel ist.

13. Medizinische Vorrichtung nach einem der Ansprüche 1 bis 11, wobei die pharmazeutische Substanz aus der Gruppe ausgewählt ist, die aus Cyclosporin A, Mycophenolsäure, Mycophenolatmofetilsäure, Rapamycin, Rapamycinderivaten, Azathiopren, Tacrolimus, Tranilast, Dexamethason, Corticosteroid, Everolimus, Retinolsäure, Vitamin E, Rosglitazon, Simvastatinen, Fluvastatin, Estrogen, 17β-Estradiol, Dihydroepiandrosteron, Testosteron, Puerarin, Thrombozytenfaktor 4, basischem Fibroblasten-Wachstumsfaktor, Fibronectin, Buttersäure, Buttersäurederivaten, Paclitaxel, Paclitaxelderivaten und Probucol besteht.

14. Medizinische Vorrichtung nach Anspruch 13, wobei die pharmazeutischen Substanzen Rapamycin, Rapamycinderivate, Paclitaxel, Paclitaxelderivate und Sirolimus umfassen.

15. Medizinische Vorrichtung nach einem der Ansprüche 1 bis 13, wobei die pharmazeutischen Substanzen Cyclosporin A und Mycophenolsäure sind.

16. Medizinische Vorrichtung nach einem der Ansprüche 1 bis 13, wobei die pharmazeutischen Substanzen Mycophenolsäure und Vitamin E sind.

17. Medizinische Vorrichtung nach einem der Ansprüche 1 bis 16, wobei die pharmazeutische Substanz etwa 1 bis etwa 50% (w/w) der Zusammensetzung ausmacht.

18. Medizinische Vorrichtung nach Anspruch 10, wobei das Poly(DL-lactid)-Polymer etwa 50 bis etwa 99% der Zusammensetzung ausmacht.

19. Medizinische Vorrichtung nach einem der Ansprüche 1 bis 18, ferner umfassend ein nicht absorbierbares Polymer.

20. Medizinische Vorrichtung nach Anspruch 19, wobei das nicht absorbierbare Polymer Methylmethacrylat ist.

21. Medizinische Vorrichtung nach Anspruch 1, wobei die Beschichtung eine einzelne homogene Schicht aus Poly(DL-lactid)-Polymer oder Poly(lactid-co-glycolid) und den pharmazeutischen Substanzen umfasst.

22. Medizinische Vorrichtung nach Anspruch 1, wobei die Beschichtung mehrere Schichten aus Poly(DL-lactid)-Polymer oder Poly(lactid-co-glycolid)-Copolymer oder einer Mischung davon umfasst.

23. Medizinische Vorrichtung nach Anspruch 1, wobei die Beschichtung mehrere Schichten aus den pharmazeutischen Substanzen umfasst.

24. Medizinische Vorrichtung nach einem der Ansprüche 1 bis 23, wobei der Ligand an der mit Blut in Kontakt kommenden Oberfläche der medizinischen Vorrichtung haftet.

25. Medizinische Vorrichtung nach einem der Ansprüche 1 bis 24, wobei der Ligand ein Antikörper oder ein Peptid ist, der oder das an ein Vorläuferzellen-Oberflächenantigen bindet.

26. Medizinische Vorrichtung nach Anspruch 1, wobei die Polymermatrix Poly(DL-co-glycolid) im Verhältnis 50:50 mit einem Molekulargewicht von 75.000 bis 100.000 ist.

27. Verfahren zum Beschichten der medizinischen Vorrichtung nach Anspruch 1, umfassend:
Aufbringen von wenigstens einer Schicht aus einer Zusammensetzung, die eine Polymermatrix mit kontrollierter Freisetzung, wenigstens eine pharmazeutische Substanz und optional eine Basalmembrankomponente umfasst, auf die Oberfläche der medizinischen Vorrichtung;
Aufbringen einer Lösung, die wenigstens einen Ligandentyp zum Binden und Immobilisieren von endothelialen Vorläuferzellen umfasst, auf die wenigstens eine Schicht aus der Zusammensetzung auf der medizinischen Vorrichtung; und
Trocknen der Beschichtung auf der medizinischen Vorrichtung unter Vakuum bei niedrigen Temperaturen.

## Revendications

1. Un dispositif médical comprenant une surface en contact avec le sang ou une surface luminale et un revêtement pour la libération contrôlée d'une ou plusieurs substances médicales dans un tissu adjacent, comprenant une ou plusieurs couches d'une matrice biocompatible bio-absorbable ou non-absorbable, comprenant une substance pharmaceutique qui empêche la migration ou la prolifération de cellules de muscles lisses, et d'un ou plusieurs ligands choisis parmi un anticorps, un fragment d'anticorps, et des combinaisons de ceux-ci, lesdits ligands étant liés par covalence à ladite matrice et configurés pour se fixer *in situ* aux antigènes de surface ou aux cellules ou aux molécules de membrane cellulaire des cellules souches endothéliales en circulation dans le sang périphérique pour la capture et la prolifération *in vivo* sur la surface en contact avec le sang du dispositif médical.

2. Le dispositif médical selon la revendication 1, où le dispositif est structuré et configuré pour être implanté dans un patient, et où au moins une surface du dispositif comprend une ou plusieurs substances de base.

3. Le dispositif médical selon la revendication 1, où le dispositif médical est une endoprothèse, un implant vasculaire ou tout autre greffon synthétique, ou une endoprothèse associée à un greffon synthétique.

4. Le dispositif médical selon la revendication 1, où le dispositif médical est une endoprothèse vasculaire.

5. L'endoprothèse vasculaire selon la revendication 4, où la structure de l'endoprothèse est constituée d'un matériau biodégradable.

6. L'endoprothèse selon la revendication 5, où le matériau biodégradable libère progressivement ladite substance pharmaceutique qui empêche la migration ou la prolifération de cellules de muscles lisses dans les tissus vasculaires adjacents.

7. Le dispositif médical selon la revendication 2, où le matériau de base est biocompatible.

8. Le dispositif médical selon la revendication 2, où le matériau de base est choisi dans le groupe comprenant l'acier inoxydable, le Nitinol, le MP35N, l'or, le tantale, le platine ou le platine iridié, des métaux et/ou alliages biocompatibles, la fibre de carbone, l'acétate de cellulose, le nitrate de cellulose, le silicone, l'hydrogel de polyvinyle d'acétate (PVA) réticulé, la mousse d'hydrogel de PVA réticulé, le polyuréthane, le polyamide, le copolymère séquencé de styrène-isobutylène-styrène (Kraton), le polyéthylène téréphtalate, le polyester, le polyorthoester, le polyanhydride, le polyéthersulfone, le polycarbonate, le polypropylène, le polyéthylène à poids moléculaire élevé, le polytétrafluoroéthylène, les polyesthers d'acide polylactique, l'acide polyglycolique, des copolymères de ceux-ci, un polyanhydride, la polycaprolactone, le valérate de polyhydroxybutyrate, des composants de matrice extracellulaire, des protéines, l'élastine, le collagène, la fibrine et des mélanges de ceux-ci.

9. Le dispositif médical selon la revendication 1, où la matrice bioabsorbable comprend un ou plusieurs polymères ou oligomères choisis parmi le groupe comprenant le polylactide-co-glycolide, l'acide polylactique, l'acide polyglycolique, un polyanhydride, la polycaprolactone, le valérate de polyhydroxybutyrate, les copolymères et des combinaisons de ceux-ci.

10. Le dispositif médical selon la revendication 1, où le revêtement comprend du poly(DL-lactide-polyglycolique) et une ou plusieurs substances pharmaceutiques.

11. Le dispositif médical selon la revendication 9, où la matrice bio-absorbable comprend du poly(DL-lactide).

12. Le dispositif médical selon la revendication 9, où la matrice bio-absorbable comprend du poly(DL-lactide) ou du poly(lactide-co-glycolide) et la substance pharmaceutique est du paclitaxel.

13. Le dispositif médical selon n'importe laquelle des revendications 1 à 11, où la substance pharmaceutique est choisie parmi le groupe comprenant la cyclosporine A, l'acide mycophénolique, l'acide mycophénolate mofétil, la rapamycine, des dérivés de la rapamycine, l'aziathioprine, le tacrolimus, le tranilast, le tranilast, la dexamétasone, les corticostéroïdes, l'évérolimus, l'acide rétinoïque, la vitamine E, la rosiglitazone, la simvastatine, la fluvastatine, les oestrogènes, le 17-bêta-oestradiol, la déhydroépiandrostérone, la testostérone, la puérarine, le facteur 4 plaquettaire, le facteur de croissance fibroblastique basique, la fibronectine, l'acide butyrique, des dérivés de l'acide butyrique, le paclitaxel, des dérivés du paclitaxel et le probucol.

14. Le dispositif selon la revendication 13, où les substances pharmaceutiques comprennent la rapamycine, des dérivés de la rapamycine, le paclitaxel, des dérivés du paclitaxel et le sirolimus.

15. Le dispositif médical de n'importe laquelle des revendications 1 à 13, dans lequel les substances pharmaceutiques sont la cyclosporine A et l'acide mycophénolique.

16. Le dispositif médical selon n'importe laquelle des revendications 1 à 13, où les substances pharmaceutiques sont l'acide mycophénolique et la vitamine E.

17. Le dispositif médical selon n'importe laquelle des revendications 1 à 16, où la substance pharmaceutique représente entre environ 1 et 50 % (p/p) de la composition.

18. Le dispositif médical selon la revendication 10, où le polymère poly(DL-lactide) représente entre environ 50 et 99 % de la composition.

19. Le dispositif médical selon n'importe laquelle des revendications 1 à 18, qui comprend en plus un polymère non-absorbable.

20. Le dispositif médical selon la revendication 19, où le polymère non-absorbable est du méthacrylate de méthyle.

21. Le dispositif médical selon la revendication 1, où le revêtement comprend une unique couche homogène de polymère de poly(DL-lactide) ou de poly(lactide-co-glycolide) et les substances pharmaceutiques.

22. Le dispositif médical selon la revendication 1, où le revêtement comprend de multiples couches de polymère de poly(DL-lactide) ou de copolymère de poly(lactide-co-glycolide), ou un mélange de ceux-ci.

23. Le dispositif médical selon la revendication 1, où le revêtement comprend de multiples couches des substances pharmaceutiques.

24. Le dispositif médical selon n'importe laquelle des revendications 1 à 23, où le ligand est lié à la surface en contact avec le sang du dispositif médical.

25. Le dispositif médical selon n'importe laquelle des revendications 1 à 24 où le ligand est un anticorps ou un peptide qui se fixe à un antigène de surface de cellules souches.

26. Le dispositif médical selon la revendication 1, où la matrice du polymère est du poly(DL-lactide-co-glycolide) dans un rapport de 50:50 dont la masse moléculaire est de 75 000 à 100 000.

27. Une méthode de revêtement du dispositif selon la revendication 1, comprenant :
l'application sur la surface dudit dispositif médical d'au moins une couche d'une composition comprenant une matrice de polymère à libération contrôlée, au moins une substance pharmaceutique, et facultativement un composant de membrane basale ;
l'application à ladite ou lesdites couche(s) de ladite composition sur ledit dispositif médical d'une solution comprenant au moins un type de ligand pour fixer et immobiliser les cellules souches endothéliales ; et
le séchage sous vide dudit revêtement sur le dispositif médical à de basses températures.
